(19)

Europäisches Patentamt
European Patent Office
Office européen des brevets

(11) **EP 4 730 357 A1**

(12) **EUROPEAN PATENT APPLICATION**
published in accordance with Art. 153(4) EPC

(43) Date of publication:
**22.04.2026  Bulletin 2026/17**

(21) Application number: **24915247.1**

(22) Date of filing: **31.12.2024**

(51) International Patent Classification (IPC):
**G16H 50/30** (2018.01)     **A61B 5/0205** (2006.01)

(52) Cooperative Patent Classification (CPC):
**A61B 5/0205; G06F 3/0481; G06F 9/451;**
**G16H 10/60; G16H 50/20; G16H 50/30;**
**G16H 50/70; G16H 80/00**

(86) International application number:
**PCT/CN2024/144183**

(87) International publication number:
**WO 2025/146033 (10.07.2025 Gazette 2025/28)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority:  **04.01.2024  CN 202410015268**

(71) Applicant: **Huawei Technologies Co., Ltd.**
**Shenzhen, Guangdong 518129 (CN)**

(72) Inventors:
• **ZHANG, Hao**
  **Shenzhen, Guangdong 518129 (CN)**
• **ZHANG, Anqi**
  **Shenzhen, Guangdong 518129 (CN)**
• **LI, Jing**
  **Shenzhen, Guangdong 518129 (CN)**

(74) Representative: **Grünecker Patent- und**
**Rechtsanwälte**
**PartG mbB**
**Leopoldstraße 4**
**80802 München (DE)**

(54) **OVARIAN HEALTH ASSESSMENT METHOD AND RELATED DEVICE**

(57)     An ovarian health evaluation method and a related device are provided, and are applied to a first electronic device. The first electronic device may display a first interface, where the first interface includes a first control; receive a first operation performed on the first control; obtain first data, where the first data includes heart rate variability data of a user within a preset time period; determine an ovarian health evaluation result based on user vital sign data, where the user vital sign data includes the first data, and the ovarian health evaluation result indicates an ovarian health status of the user within the preset time period; and display a second interface after the preset time period, where display content of the second interface includes the ovarian health evaluation result. According to the method and the device, an abnormal ovarian function of the user can be detected in a timely manner.

First electronic device

Ovarian health evaluation

21

Detection start

211

FIG. 3b

## Description

This application claims priority to Chinese Patent Application No. 202410015268.6, filed with the China National Intellectual Property Administration on January 4, 2024 and entitled "OVARIAN HEALTH EVALUATION METHOD AND RELATED DEVICE", which is incorporated herein by reference in its entirety.

## TECHNICAL FIELD

This application relates to the field of intelligent terminal technologies, and in particular, to an ovarian health evaluation method and a related device.

## BACKGROUND

An ovary is both a reproductive organ and an endocrine organ. Two main functions of the ovary are to produce eggs and secrete sex hormones (mainly estrogen and progesterone), to ensure normal growth and development of women, and maintain health of the women and normal fertility. Ovarian aging is a pacemaker of female body aging and the ovary is a first organ that ages comprehensively in a female body. Ovarian aging may cause a plurality of other organs in the female body to age, leading to an increasing incidence of osteoporosis, cardiovascular diseases, senile dementia, obesity, diabetes, and other diseases, and a decrease in the fertility and fertility quality.

The ovarian functions decline until fail, which is an inevitable process. Currently, an average menopause age of the women is 51 years old. With extension of life, increasing women spend nearly one third of their lives after menopause. According to statistics, there are 130 million women in the perimenopausal period in China, and the quantity is expected to exceed 280 million by 2030. A quantity of women in the perimenopausal period will increase to 1.2 billion worldwide. Due to genetic, behavioral, psychological, immune, and other reasons, or because other diseases, especially malignant tumors, need to be treated, ovarian functions of some people are damaged to different degrees, resulting in an excessively rapid ovarian function decline. As a result, ovarian dysfunction diseases such as early onset ovarian insufficiency, premature ovarian failure, and an ovarian reserve function decline occur. In recent years, an incidence of ovarian function decline diseases has gradually increased and a younger trend is shown. If a symptom of abnormal ovarian aging can be detected early, a related symptom caused by the ovarian aging can be improved.

Currently, ovarian health evaluation basically depends on professional medical means such as blood hormone detection, ovarian biopsy sampling, ovarian CT, and color ultrasound examination. The foregoing ovarian health evaluation method is costly and inconvenient, and cannot meet a requirement of a user for daily ovarian health evaluation.

## SUMMARY

Embodiments of this application provide an ovarian health evaluation method and a related device, so that an abnormal ovarian function of a user can be detected in a timely manner.

According to a first aspect, an embodiment of this application provides an ovarian health evaluation method, applied to a first electronic device, where the method includes: displaying a first interface, where the first interface includes a first control; receiving a first operation performed on the first control; obtaining first data, where the first data includes heart rate variability data of a user within a preset time period; determining an ovarian health evaluation result based on user vital sign data, where the user vital sign data includes the first data; and the ovarian health evaluation result indicates an ovarian health status of the user within the preset time period; and displaying a second interface after the preset time period, where display content of the second interface includes the ovarian health evaluation result.

In conclusion, in this embodiment of this application, the first electronic device may receive the first operation (for example, a touch operation) performed by the user on a detection start control (namely, the first control) of a detection start interface (namely, the first interface), and obtain the first data. The first data includes at least the heart rate variability data of the user within the preset time period. The user vital sign data may include the first data. The user vital sign data may be used as a basis for evaluating the ovarian health of the user, and the ovarian health evaluation result may be determined based on the user vital sign data. In this application, the ovarian health of the user can be evaluated based on the heart rate variability data of the user, and the ovarian health of the user does not need to be evaluated through endocrine detection, so that a portable and low-cost ovarian health evaluation solution is provided. Further, after the preset time period, a detection result interface may be displayed on the first electronic device, and display content of the detection result interface includes the ovarian health evaluation result. The ovarian health evaluation result of the user is displayed on the first electronic device, so that the abnormal ovarian function of the user can be detected in a timely manner. If a symptom of abnormal ovarian aging can be detected in a timely manner, a related symptom caused by ovarian aging can be improved.

In some embodiments, the first electronic device is connected to N second electronic devices, and N is an integer greater than 0; and after the first electronic device receives the first operation performed on the first control, the method further includes: receiving second data sent by M second electronic devices in the N second electronic devices, where the user vital sign data further includes the second data.

In this embodiment of this application, when the first electronic device is connected to the N second electronic devices, the first electronic device may further obtain the second data from a part or all of the N second electronic devices. In this way, the user vital sign data may include the first data and the second data. The user vital sign data may be used as a basis for evaluating the ovarian health of the user, that is, the ovarian health evaluation result may be determined based on the user vital sign data. In this application, an advantage of multi-device collaboration can be fully used, and a plurality of intelligent devices can be collaborated to provide more comprehensive monitoring of various body indicators, to implement multi-dimensional and comprehensive ovarian health evaluation, so as to improve accuracy of the ovarian health evaluation result.

In some embodiments, the second data includes one or more of the following: blood pressure data of the user within the preset time period, body fat data of the user within the preset time period, emotion data of the user within the preset time period, and skin temperature data of the user within the preset time period.

In this embodiment of this application, the second data includes one or more of the blood pressure data of the user within the preset time period, the body fat data of the user within the preset time period, the emotion data of the user within the preset time period, and the skin temperature data of the user within the preset time period. In this way, the user vital sign data may include more data related to the user vital sign. The user vital sign data may be used as a basis for evaluating the ovarian health of the user, that is, the ovarian health evaluation result may be determined based on the user vital sign data. In this application, an advantage of multi-device collaboration can be fully used, and a plurality of intelligent devices can be collaborated to provide more comprehensive monitoring of various body indicators, to implement multi-dimensional and comprehensive ovarian health evaluation, so as to improve accuracy of the ovarian health evaluation result.

In some embodiments, the method further includes: displaying a third interface, where display content of the third interface includes a user questionnaire survey question; and receiving an input operation performed by the user on the third interface, and determining a questionnaire survey result, where the user vital sign data further includes questionnaire survey data.

In this embodiment of this application, the first electronic device may further display a user questionnaire survey interface (namely, the third interface), and the user may input the questionnaire survey result on the user questionnaire survey interface. The first electronic device may receive an input operation performed by the user on the user questionnaire survey interface, and may further determine the ovarian health evaluation result based on the user vital sign data and user information (that is, the questionnaire survey result) input by the user. Questionnaire survey content may include but is not limited to an osteoporosis symptom, an emotional status, and the like. In this application, data of the user in more dimensions is collected with reference to the questionnaire survey, including but not limited to the osteoporosis symptom, the emotional status, and the like, and the data may be jointly used as a basis for evaluating the ovarian health, to implement multi-dimensional and comprehensive ovarian health evaluation, so as to improve accuracy of the ovarian health evaluation result.

In some embodiments, determining the ovarian health evaluation result based on the user vital sign data includes: obtaining user information, where the user information includes one or more of medication, drinking, a disease history, and a sleep environment temperature of the user; and determining the ovarian health evaluation result based on the user information and the user vital sign data.

In this embodiment of this application, user information may be obtained, and the user information may include but is not limited to information such as the medication, the drinking, the disease history, and the sleep environment temperature of the user. Optionally, the user vital sign data may be preprocessed based on the user information, to determine the preprocessed user vital sign data, and the ovarian health of the user may further be evaluated based on the preprocessed user vital sign data. In this embodiment of this application, considering that a part of the obtained user vital sign data may have an abnormal fluctuation, if the data is directly used, accuracy of subsequent feature extraction may be reduced, affecting accuracy of ovarian function evaluation. Therefore, data denoising and interference removal operations may be performed on the user vital sign data based on the user information, to effectively improve accuracy of feature extraction, so as to improve accuracy of ovarian health evaluation. Optionally, user information may also be obtained, and the ovarian health evaluation result is determined based on the user information and the user vital sign data, to implement multi-dimensional and comprehensive ovarian health evaluation, and improve accuracy of the ovarian health evaluation result.

In some embodiments, the first data further includes one or more of the following: heart rate data of the user within the preset time period, respiratory rate data of the user within the preset time period, and the skin temperature data of the user within the preset time period. The method further includes: obtaining one or more of the heart rate data, the respiratory rate data, and the skin temperature data; displaying a fourth interface when one or more of the heart rate data, the respiratory rate data, and the skin temperature data meet a first condition, where the fourth interface includes a third control and a fourth control, the third control is used by the user to confirm that a hot flash currently occurs, and the fourth control is used by the user to confirm that no hot flash currently occurs; and receiving an operation performed by the user on the third control or the fourth control.

In this embodiment of this application, when one or more of the heart rate data, the respiratory rate data, and the skin temperature data meet the first condition, it may be determined that the user has a hot flash, so that the first electronic device may display a hot flash confirmation interface, namely, the fourth interface. The hot flash confirmation interface includes two preset controls: the third control and the fourth control. The third control may be used by the user to confirm that a hot flash currently occurs, and the fourth control is used by the user to confirm that no hot flash currently occurs. The first electronic device receives an input operation performed by the user on the hot flash confirmation interface. In this embodiment of this application, a user positive feedback mechanism may be designed. For example, when a hot flash is detected by using a hot flash detection algorithm, the user may be prompted to confirm whether the hot flash occurs, to optimize the ovarian evaluation result based on the user hot flash data, so as to improve accuracy of ovarian function evaluation and improve individual interaction experience of the user.

In some embodiments, the first condition is that a data change amplitude of one or more of the heart rate data, the respiratory rate data, and the skin temperature data in a first time interval within the preset time period is greater than or equal to a first preset threshold.

In this embodiment of this application, the first time interval is a time interval within the preset time period, for example, 5 minutes. When it is detected that one or more of the heart rate data, the respiratory rate data, and the skin temperature data of the user fluctuates greatly in a short time, it may be determined that the user has a hot flash.

In some embodiments, determining the ovarian health evaluation result based on the user vital sign data includes: determining target data based on the user vital sign data; and determining the ovarian health evaluation result based on the target data and an ovarian function evaluation model.

In this embodiment of this application, the target data may be input data input into the ovarian function evaluation model. The user vital sign data may be first processed, and then the processed data may be input into the ovarian function evaluation model, so that the ovarian health evaluation result can be determined, and the ovarian health of the user does not need to be evaluated based on the endocrine detection, to provide a portable and low-cost ovarian health evaluation solution.

In some embodiments, determining the target data based on the user vital sign data includes: determining symptom information of the user based on the user vital sign data, where the symptom information includes a hot flash frequency and an autonomic nerve function level of the user within the preset time period; and determining the target data based on the symptom information.

In this embodiment of this application, the user symptom indicator (which may be used as symptom information) may be first determined based on a plurality of vital sign indicators included in the user vital sign data.

For example, a hot flash frequency of the user and an autonomic nerve function level may be detected based on the vital sign indicators in the user vital sign data. Further, the target data may be determined based on indicators included in the user symptom indicator. In this embodiment of this application, original input data is converted into symptoms in different dimensions caused by an ovarian function decline, and a feature that can be directly used for ovarian function evaluation is determined, so that the ovarian health of the user can be evaluated more accurately.

In some embodiments, the method further includes: obtaining user health check data, where the user health check data includes a user anti-mullerian hormone test result and/or a user follicle-stimulating hormone test result; and adjusting a parameter in the ovarian function evaluation model based on the user health check data.

In this embodiment of this application, user health check data may be obtained, where the user health check data may include but is not limited to an anti-mullerian hormone (Anti-Mullerian hormone, AMH) test result and a follicle-stimulating hormone (Follicle-stimulating hormone, FSH) test result. The ovarian function evaluation model may be optimized based on the user health check data, for example, a parameter threshold in the ovarian function evaluation model may be adjusted. In this embodiment of this application, corresponding hormone level information of the user is obtained, and an ovarian health degree corresponding to the hormone level is used as a golden standard of a current ovarian health degree of the user, so that the parameter threshold in the ovarian function evaluation model can be optimized, and the like, to improve accuracy of subsequent detection.

In some embodiments, the first data further includes a sleep parameter of the user within the preset time period.

In this embodiment of this application, the first data further includes the sleep parameter of the user within the preset time period, to implement multi-dimensional and comprehensive ovarian health evaluation, so as to improve accuracy of the ovarian health evaluation result.

In some embodiments, the N second electronic devices include a smart speaker, and the method further includes: determining, based on the sleep parameter within the preset time period, whether sleep quality of the user is less than a second preset threshold; and if the sleep quality is less than the second preset threshold, sending first information to the smart speaker, where the first information indicates the smart speaker to play a target audio.

In this embodiment of this application, the first electronic device may further collaborate with a smart home to adjust a user space environment. For example, when it is detected that sleep quality is poor, the first electronic device may collaborate with a smart speaker to play soothing music before the user sleeps/during sleeping of the user, to improve the sleep quality.

In some embodiments, the N second electronic devices include a smart air conditioner, and the method further includes: predicting, within the preset time period, a target moment at which the user has a hot flash, and sending second information to the smart air conditioner before the target moment, where the second information indicates the smart air conditioner to adjust to a target temperature.

In this embodiment of this application, the first electronic device may further collaborate with the smart home to adjust the user space environment, for example, intelligently adjust an air conditioner temperature when it is predicted that the hot flash occurs, to alleviate a hot flash symptom of the user.

In some embodiments, the method may further include: uploading the ovarian health evaluation result to a cloud server, where the cloud server is configured to: determine care prompt information based on the ovarian health evaluation result, and send the care prompt information to a third electronic device, where the third electronic device and the first electronic device are bound as a first relationship; and the third electronic device is configured to display a fifth interface, where display content of the fifth interface includes the care prompt information.

In this embodiment of this application, the first electronic device may further collaborate with a family device (namely, the third electronic device) to perform relative or friend care. For example, the first electronic device pushes, to a relative or friend, information indicating that physical discomfort, an emotional fluctuation, and the like may occur on the user, so that the relative or friend makes preparations in advance, provides more care, and avoids an emotional conflict, to reduce user discomfort.

According to a second aspect, an electronic device is provided, including a memory and one or more processors. The memory is coupled to the one or more processors, the memory is configured to store computer program code, the computer program code includes computer instructions, and the one or more processors invoke the computer instructions, so that the electronic device is enabled to perform the method according to the first aspect or any implementation of the first aspect.

According to a third aspect, a computer-readable storage medium is provided, including instructions. When the instructions are run on an electronic device, the electronic device is enabled to perform the method according to the first aspect or any implementation of the first aspect.

According to a fourth aspect, a computer program product is provided, and when the computer program product runs on a computer, the computer is enabled to perform the method according to the first aspect or any implementation of the first aspect.

According to a fifth aspect, this application provides a chip system. The chip system includes at least one processor, configured to implement the method according to the first aspect or any implementation of the first aspect.

**BRIEF DESCRIPTION OF DRAWINGS**

FIG. 1a is a diagram of a structure of an electronic device according to an embodiment of this application;
FIG. 1b is a block diagram of a software structure of an electronic device 100 according to an embodiment of this application;
FIG. 1c is a block diagram of another software structure of an electronic device 100 according to an embodiment of this application;
FIG. 2 is a diagram of an ovarian health evaluation system according to an embodiment of this application;
FIG. 3a to FIG. 3i are diagrams of a group of user interfaces displayed on a first electronic device according to an embodiment of this application;
FIG. 4a to FIG. 4e(b) are diagrams of a group of user interfaces displayed on a second electronic device according to an embodiment of this application;
FIG. 5 is a diagram of a user interface displayed on a third electronic device according to an embodiment of this application;
FIG. 6A to FIG. 6C are a schematic flowchart of an ovarian health evaluation method according to an embodiment of this application;
FIG. 7 is a schematic flowchart of another ovarian health evaluation method according to an embodiment of this application; and
FIG. 8 is a diagram of an electronic device according to an embodiment of this application.

**DESCRIPTION OF EMBODIMENTS**

The following describes embodiments of this application with reference to the accompanying drawings in embodiments of this application.

In the specification, claims, and accompanying drawings of this application, the terms "first", "second", "third", "fourth" and so on are intended to distinguish between different objects but do not indicate a particular order. In addition, terms such as "include" and "have" and any other variants thereof are intended to cover a non-exclusive inclusion. For example, a process, a method, a system, a product, or a device that includes a series of steps or units is not limited to the listed steps or units, but optionally further includes an unlisted step or unit, or optionally further includes another inherent step or unit of the process, the method, the product, or the device.

"Embodiments" mentioned in this specification mean that specific features, structures, or characteristics described in combination with embodiments may be included in at least one embodiment of this application. The phrase shown in various locations in the specification may not necessarily refer to a same embodiment, and is not an independent or optional embodiment exclusive from another embodiment. It is explicitly and implicitly understood by a person skilled in the art that embodiments described in this specification may be combined with other embodiments.

An ovarian health evaluation method provided in this embodiment of this application may be applied to an electronic device. Currently, some electronic devices are, for example, a mobile phone (mobile phone), a tablet computer, a notebook computer, a palmtop computer, a mobile internet device (mobile internet device, MID), a wearable device (for example, a smartwatch), a virtual reality (virtual reality, VR) device, an augmented reality (augmented reality, AR) device, a wireless terminal in industrial control (industrial control), a wireless terminal in self driving (self driving), a wireless terminal in remote medical surgery (remote medical surgery), a wireless terminal in a smart grid (smart grid), a wireless terminal in transportation safety (transportation safety), a wireless terminal in a smart city (smart city), a wireless terminal in a smart home (smart home), a cellular phone, a cordless phone, a session initiation protocol (session initiation protocol, SIP) phone, a wireless local loop (wireless local loop, WLL) station, a personal digital assistant (personal digital assistant, PDA), a hand-held device or a computing device that has a wireless communication function or another processing device connected to a wireless modem, a vehicle-mounted device, a wearable device, a terminal device in a 5G network, or a terminal device in a future evolved public land mobile network (public land mobile network, PLMN). This is not limited in embodiments of this application.

Before the method provided in embodiments of this application is described in detail, the electronic device provided in embodiments of this application is first described.

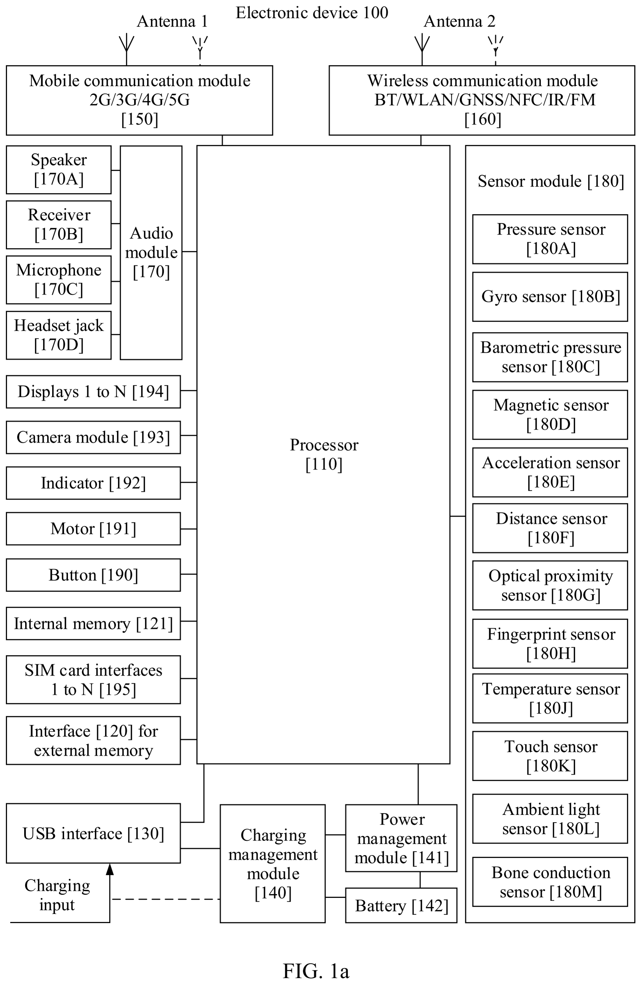

FIG. 1a is a diagram of a structure of an electronic device 100.

The electronic device 100 may include a processor 110, an interface 120 for external memory, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera module 193, a display 194, a subscriber identification module (subscriber identification module, SIM) card interface 195, and the like.

The sensor module 180 may include a pressure sensor 180A, a gyro sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like. The sensor module 180 may further include an optical heart rate sensor that is not shown in FIG. 1a.

The pressure sensor 180A is configured to sense a pressure signal, and can convert the pressure signal into an electrical signal. In some embodiments, the pressure sensor 180A may be disposed on the display 194. There are a plurality of types of pressure sensors 180A, such as a resistive pressure sensor, an inductive pressure sensor, and a capacitive pressure sensor. The capacitive pressure sensor may include at least two parallel plates made of conductive materials. When a force acts on the pressure sensor 180A, capacitance between electrodes changes. The electronic device 100 determines pressure intensity based on the change in the capacitance. When a touch operation is performed on the display 194, the electronic device 100 detects intensity of the touch operation through the pressure sensor 180A. The electronic device 100 may also calculate a touch location based on a detection signal of the pressure sensor 180A. In some embodiments, touch operations that are performed in a same touch location but have different touch operation intensity may correspond to different operation instructions. For example, when a touch operation whose touch operation intensity is less than a first pressure threshold is performed on a Messages application icon, an instruction for viewing an SMS message is performed. When a touch operation whose touch operation intensity is greater than or equal to the first pressure threshold is performed on the Messages application icon, an instruction for creating a new SMS message is performed.

The gyro sensor 180B may be configured to determine a motion posture of the electronic device 100. In some embodiments, an angular velocity of the electronic device 100 around three axes (axes x, y, and z) may be determined through the gyro sensor 180B. The gyro sensor 180B may be configured to implement image shooting stabilization during image shooting. For example, when the shutter is pressed, the gyro sensor 180B detects an angle at which the electronic device 100 jitters, calculates, based on the angle, a distance for which a lens module needs to compensate, and allows the lens to counteract the shake of the electronic device 100 through reverse motion, to implement stabilization. The gyro sensor 180B may also be used in a navigation scenario and a somatic game scenario.

The barometric pressure sensor 180C is configured to measure barometric pressure. In some embodiments, the electronic device 100 calculates an altitude through the barometric pressure measured by the barometric pressure sensor 180C, to assist in positioning and navigation.

The magnetic sensor 180D includes a Hall effect sensor. The electronic device 100 may detect opening and closing of a flip cover through the magnetic sensor 180D. In some embodiments, when the electronic device 100 is a clamshell phone, the electronic device 100 may detect opening and closing of a flip cover through the magnetic sensor 180D. Further, a feature like automatic unlocking of the flip cover is set based on a detected opening or closing state of the flip cover.

The acceleration sensor 180E may detect accelerations in various directions (usually on three axes) of the electronic device 100. When the electronic device 100 is still, a magnitude and a direction of gravity may be detected. The acceleration sensor may be further configured to identify a posture of the electronic device 100, and is used in switching between a landscape mode and a portrait mode, a pedometer, or another application.

The distance sensor 180F is configured to measure a distance. The electronic device 100 may measure the distance in an infrared manner or a laser manner. In some embodiments, in an image shooting scene, the electronic device 100 may measure a distance by using the distance sensor 180F, to implement quick focusing.

The optical proximity sensor 180G may include, for example, a light-emitting diode (LED) and an optical detector, for example, a photodiode. The light-emitting diode may be an infrared light-emitting diode. The electronic device 100 emits infrared light through the light-emitting diode. The electronic device 100 detects infrared reflected light from a nearby object through the photodiode. When sufficient reflected light is detected, it may be determined that there is an object near the electronic device 100. When insufficient reflected light is detected, the electronic device 100 may determine that there is no object near the electronic device 100. The electronic device 100 may detect, by using the optical proximity sensor 180G, that the user holds the electronic device 100 close to an ear for a call, to automatically perform screen-off for power saving. The optical proximity sensor 180G may also be used in a leather case mode or a pocket mode to automatically perform screen unlocking or locking.

The ambient light sensor 180L is configured to sense ambient light brightness. The electronic device 100 may adaptively adjust brightness of the display 194 based on the sensed ambient light brightness. The ambient light sensor 180L may also be configured to automatically adjust white balance during image shooting. The ambient light sensor 180L may also cooperate with the optical proximity sensor 180G, to detect whether the electronic device 100 is in a pocket, to avoid an accidental touch.

The fingerprint sensor 180H is configured to collect a fingerprint. The electronic device 100 may use a feature of the collected fingerprint to implement fingerprint-based unlocking, application lock access, fingerprint-based image shooting, fingerprint-based call answering, and the like.

The temperature sensor 180J is configured to detect a temperature. In some embodiments, the electronic device 100 executes a temperature processing policy by utilizing the temperature detected by the temperature sensor 180J. For example, when the temperature reported by the temperature sensor 180J exceeds a threshold, the electronic device 100 lowers performance of a processor nearby the temperature sensor 180J, to reduce power consumption for thermal protection. In some other embodiments, when the temperature is less than another threshold, the electronic device 100 heats the battery 142 to prevent the electronic device 100 from being shut down abnormally due to a low temperature. In some other embodiments, when the temperature is less than still another threshold, the electronic device 100 boosts an output voltage of the battery 142 to avoid abnormal shutdown caused by a low temperature. In this embodiment of this application, the temperature sensor 180J may detect a skin temperature of the user, to obtain skin temperature data of the user.

The touch sensor 180K is also referred to as a "touch panel". The touch sensor 180K may be disposed on the display 194, and the touch sensor 180K and the display 194 form a touchscreen, which is also referred to as a "touch screen". The touch sensor 180K is configured to detect a touch operation performed on or near the touch sensor. The touch sensor may transfer the detected touch operation to the application processor to determine a type of the touch event. A visual output related to the touch operation may be provided on the display 194. In some other embodiments, the touch sensor 180K may also be disposed on a surface of the electronic device 100 at a location different from that of the display 194.

The bone conduction sensor 180M may obtain a vibration signal. In some embodiments, the bone conduction sensor 180M may obtain a vibration signal of a vibration bone of a human vocal-cord part. The bone conduction sensor 180M may also be in contact with a body pulse, to receive a blood pressure beating signal. In some embodiments, the bone conduction sensor 180M may alternatively be disposed in the headset, to combine into a bone conduction headset. The audio module 170 may obtain a speech signal through parsing based on the vibration signal that is of the vibration bone of the vocal-cord part and that is obtained by the bone conduction sensor 180M, to implement a speech function. The application processor may parse heart rate information based on the blood pressure pulse signal obtained by the bone conduction sensor 180M, to implement a heart rate detection function.

The optical heart rate sensor is one of popular sensors used for heart rate detection in a smart wearable device. The optical heart rate sensor adopts a photoplethysmography (PPG) to measure the heart rate and other biometric indicators. Measurement principle: Capacitive light is emitted to skin, light reflected by skin tissue is received by a photosensitive sensor and converted into an electrical signal. Then, the electrical signal is converted into a digital signal. The heart rate can be calculated based on a light absorption rate of blood. In this embodiment of this application, the optical heart rate sensor may detect a heart rate, a respiratory rate, heart rate variability, and the like of the user, to obtain heart rate data, respiratory rate data, heart rate variability data, and sleep status indicator data of the user.

In this embodiment of this application, the optical heart rate sensor, the acceleration sensor 180E, the gyro sensor 180B, and the like may jointly detect a sleep score, a quantity of waking-up in a sleep process, and the like of the user (that is, detect the sleep status indicator), to obtain sleep status indicator data of the user.

In some embodiments, the electronic device 100 may further include an airbag, and the airbag may assist in blood pressure monitoring.

It may be understood that the structure shown in this embodiment of this application does not constitute a specific limitation on the electronic device 100. In some other embodiments of this application, the electronic device 100 may include more or fewer components than those shown in the figure, or a combination of a part of the components, or splits from a part of the components, or an arrangement of different components. The components shown in the figure may be implemented by hardware, software, or a combination of software and hardware.

The processor 110 may include one or more processing units. For example, the processor 110 may include an application processor (application processor, AP), a central processing unit (central processing unit, CPU), a graphics processing unit (graphics processing unit, GPU), a neural-network processing unit (neural-network processing unit, NPU), a modem processor, an image signal processor (image signal processor, ISP), a controller, a memory, a video codec, a digital signal processor (digital signal processor, DSP), a baseband processor, and the like. Different processing units may be independent components, or may be integrated into one or more processors.

The controller may be a nerve center and a command center of the electronic device 100. The controller may generate an operation control signal based on instruction operation code and a time sequence signal, to complete control of instruction reading and instruction execution.

A memory may be further disposed in the processor 110, and is configured to store instructions and data. In some embodiments, the memory in the processor 110 is a cache. The memory may store instructions or data just used or cyclically used by the processor 110. If the processor 110 needs to use the instruction or the data again, the processor may directly invoke the instruction or the data from the memory. This avoids repeated access, reduces a waiting time of the processor 110, and improves efficiency of the electronic device 100.

The display 194 is configured to display a user interface, an image, a video, and the like. The display 194 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini-LED, a micro-LED, a micro-OLED, a quantum dot light-emitting diode (quantum dot light-emitting diode, QLED), or the like.

The interface 120 for external memory may be configured to connect to an external storage card, for example, a micro SD card, to extend a storage capability of the electronic device 100. The external storage card communicates with the processor 110 through the interface 120 for external memory, to implement a data storage function. For example, data such as the user vital sign data and a user image is stored in the external storage card.

The internal memory 121 may be configured to store one or more computer programs, and the one or more computer programs include instructions. The processor 110 may run the instructions stored in the internal memory 121, so that the electronic device 100 performs an ovarian health evaluation method provided in some embodiments of this application, various function applications, data processing, and the like. The internal memory 121 may include a program storage region and a data storage region. The program storage region may store an operating system. The program storage region may further store one or more applications (for example, Huawei health or Contacts), and the like. The data storage region may store data (for example, user vital sign data) created when the electronic device 100 is used, and the like. In addition, the internal memory 121 may include a high-speed random access memory, and may further include a non-volatile memory, for example, at least one magnetic disk storage component, a flash memory, or a universal flash storage (universal flash storage, UFS).

The electronic device 100 shown as an example in FIG. 1a may display, on the display 194, user interfaces described in the following embodiments. The electronic device 100 may detect, on each user interface through the touch sensor 180K, a touch operation, for example, a tap operation (like a touch operation or a double-tap operation on an icon) on each user interface, or for another example, an upward or downward sliding operation, or an operation of performing a circle drawing gesture, or the like on each user interface. In some embodiments, the electronic device 100 may detect, through the gyro sensor 180B, the acceleration sensor 180E, or the like, a motion gesture performed by the user holding the electronic device 100, for example, shaking the electronic device. In some embodiments, the electronic device 100 may detect a non-touch gesture operation through the camera module 193 (for example, a 3D camera).

A software system of the electronic device 100 may use a layered architecture, an event-driven architecture, a microkernel architecture, a micro service architecture, or a cloud architecture. In this embodiment of this application, the Android system with a layered architecture is used as an example to illustrate a software structure of the electronic device 100.

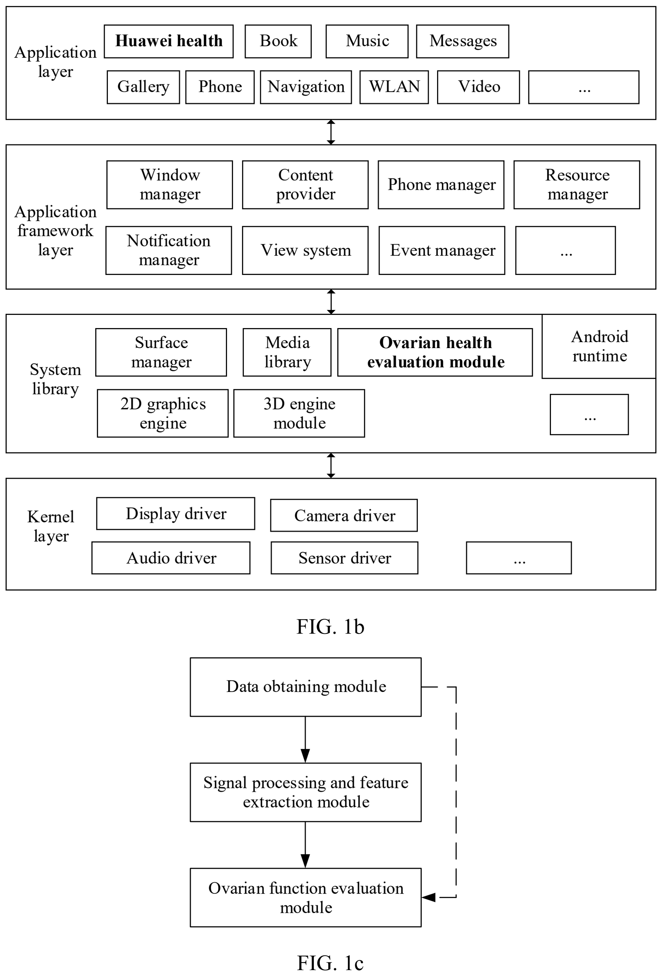

FIG. 1b is a block diagram of a software structure of an electronic device 100 according to an embodiment of this application.

In a layered architecture, software is divided into several layers, and each layer has a clear role and task. The layers communicate with each other through a software interface. In some embodiments, the Android system is divided into four layers: an application layer, an application framework layer, an Android runtime (Android runtime) and system library, and a kernel layer from top to bottom.

The application layer may include a series of application packages.

As shown in FIG. 1b, the application packages may include applications such as Huawei health, Camera, Gallery, Calendar, Phone, Navigation, WLAN, Bluetooth, Music, Video, and Messages.

The Huawei health application may provide one or more functions, and the one or more functions include at least an ovarian health evaluation function provided in this application. The function can detect an abnormal ovarian function of the user in a timely manner. The Huawei health application may receive an interaction operation performed by the user on the user interface, for example, an operation of filling in a questionnaire survey by the user, an operation of importing a check report by the user, and an operation of viewing an ovarian evaluation result by the user. The ovarian health evaluation function of the Huawei health application is clearly described herein and in subsequent method embodiments, and a name of the ovarian health evaluation function does not constitute a limitation on this application. For example, the Huawei health application may also be referred to as "Health", "Ovarian health monitoring", or the like. This is not limited herein.

The application framework layer provides an application programming interface (application programming interface, API) and a programming framework for an application at the application layer. The application framework layer includes some predefined functions.

As shown in FIG. 1b, the application framework layer may include a window manager, a content provider, a view system, a phone manager, a resource manager, and a notification manager.

The window manager is configured to manage a window program. The window manager may obtain a size of a display, determine whether there is a status bar, perform screen locking, take a screenshot, or the like.

The content provider is configured to: store and obtain data, and enable the data to be accessible to an application. The data may include a user vital sign data, an image, a video, an audio, calls that are made and answered, a browsing history and bookmarks, an address book, and the like.

The user vital sign data (which may also be referred to as user physiological data) may include but is not limited to a skin temperature, a heart rate, a respiratory rate, heart rate variability, a sleep status indicator, a blood pressure, a body fat, an emotion, and the like of the user within a preset time period.

The view system includes visual controls such as a control for displaying a text and a control for displaying an image. The view system may be used to construct an application. A display interface may include one or more views. For example, a display interface including an SMS message notification icon may include a text display view and an image display view.

The phone manager is configured to provide a communication function for the electronic device 100, for example, management of a call status (including answering, declining, or the like).

The resource manager provides various resources such as a localized image, a character string, an icon, a layout file, and a video file for an application.

The notification manager enables an application to display notification information in a status bar, and may be configured to convey a notification message, which may automatically disappear after a short pause without requiring a user interaction. For example, the notification manager is configured to notify download completion, give a message notification, and the like. The notification manager may alternatively be a notification that appears in a top status bar of the system in a form of graph or scroll bar text, for example, a notification of an application that is run on a background, or may be a notification that appears on the screen in a form of dialog window. For example, text information is prompted in the status bar, an alert tone is made, the electronic device vibrates, or the indicator blinks.

The Android runtime includes a core library and a virtual machine. The Android runtime is responsible for scheduling and management of the Android system.

The core library includes two parts: a function that needs to be invoked in a Java language and a core library of Android.

The application layer and the application framework layer run on the virtual machine. The virtual machine executes Java files of the application layer and the application framework layer as binary files. The virtual machine is configured to implement functions such as object lifecycle management, stack management, thread management, security and exception management, and garbage collection.

The system library may include a plurality of functional modules, such as a surface manager (surface manager), a media library (Media Library), a 3D engine module (for example, OpenGL ES), a 2D graphics engine (for example, SGL), and an ovarian health evaluation module.

The surface manager is configured to manage a display subsystem and provide fusion of 2D and 3D layers for a plurality of applications.

The media library supports playback and recording of a plurality of commonly used audio and video formats, static image files, and the like. The media library may support a plurality of audio and video encoding formats, for example, MPEG-4, G.264, MP3, AAC, AMR, JPG, and PNG.

The 3D engine module is configured to implement three-dimensional graphics drawing, image rendering, composition, layer processing, and the like.

The 2D graphics engine is a drawing engine for 2D drawing.

The ovarian health evaluation module is configured to: obtain the user vital sign data, and analyze and process the user vital sign data based on an ovarian evaluation model, to determine an ovarian health evaluation result, so that an abnormal ovarian function of the user can be detected in a timely manner. Further descriptions will be provided subsequently, and details are not described herein.

The kernel layer is a layer between hardware and software. The kernel layer includes at least a display driver, a camera driver, an audio driver, and a sensor driver.

FIG. 1c is a block diagram of another software structure of an electronic device 100 according to an embodiment of this application. In some embodiments, a software architecture may include a data obtaining module, a signal processing and feature extraction module, and an ovarian function evaluation module.

The data obtaining module may obtain user vital sign data by using a device like a smartwatch, a smart ring, or a smart band, for example, physiological data that can be used to determine a hot flash, and evaluate an autonomic nerve function level and a sleep disorder, for example, skin temperature data, heart rate data, respiratory rate data, heart rate variability data, and sleep status indicator data (a sleep score, quantity of waking-up, and the like). In addition, more-dimensional data of a user may be collected with reference to questionnaire survey, including but not limited to an osteoporosis symptom, an emotional status, and the like. The data obtained by the data obtaining module may be used to evaluate an ovarian function of the user.

The signal processing and feature extraction module may analyze and process the data obtained by the data obtaining module, for example, calculate a hot flash frequency of the user based on a skin temperature, a heart rate, and a respiratory rate parameter, calculate the autonomic nerve function level based on a heart rate variability-related parameter, calculate an insomnia level based on a sleep monitoring indicator, calculate a comprehensive symptom severity score based on a questionnaire input, and convert original input data into symptom features in different dimensions caused by an ovarian function decline, to determine features that can be directly used for ovarian function evaluation.

The ovarian function evaluation module may evaluate an ovarian function based on multi-dimensional features output by the signal processing and feature extraction module, to determine an ovarian health evaluation result. Optionally, the ovarian health evaluation result may be: a low risk or a high risk of the ovarian function decline. Optionally, the ovarian health evaluation result may be further distinguished into a medium risk, a high risk, and the like based on severity. The ovarian health evaluation result indicates an ovarian health status of the user within a preset time period. A form of the ovarian health evaluation result is not specifically limited in this application.

In some embodiments, a software architecture may include a data obtaining module and an ovarian function evaluation module. The data obtaining module may obtain user vital sign data through a device like a smartwatch, a smart ring, or a smart band, for example, skin temperature data, heart rate data, respiratory rate data, heart rate variability data, and sleep status indicator data (a sleep score, quantity of waking-up, and the like). The ovarian function evaluation module may directly determine an ovarian health evaluation result of a user based on the user vital sign data obtained by the data obtaining module.

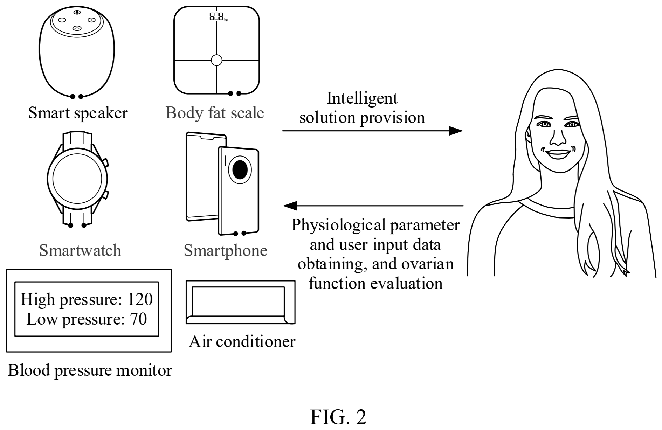

FIG. 2 is a diagram of an ovarian health evaluation system according to an embodiment of this application. The ovarian health evaluation system includes a first electronic device and N second electronic devices, where N is an integer greater than or equal to 0. The first electronic device is the electronic device 100 mentioned above, and the first electronic device may be a device that can be used to measure physiological data of a user, for example, a wearable device. For example, the first electronic device may be a smartwatch or a smart band. A device connected to the first electronic device may be referred to as a second electronic device, and each of the N second electronic devices connected to the first electronic device may also include a part or all functions of the electronic device 100. The N second electronic devices may include a terminal and/or a smart home. For example, the N second electronic devices may include a smartphone, a smart speaker, a body fat scale, an air conditioner, a blood pressure monitor, and the like. It may be understood that the first electronic device may be directly connected to the second electronic device, or may be indirectly connected to the second electronic device. It may be understood that the first electronic device may be indirectly connected to the second electronic device through another device. In this case, the first electronic device may send information to the second electronic device through the another device. The first electronic device and the second electronic device may be connected by using a wireless connection technology, or may be connected through a wired connection. The wireless connection technology may include a communication technology like a wireless communication (Wireless fidelity, Wi-Fi) technology, a Bluetooth (Bluetooth) technology, a ZigBee (ZigBee) technology, or a Near Link (Near Link) technology. This is not specifically limited in this application. It should be noted that connection manners between the first electronic device and different second electronic devices may be the same or may be different. This is not specifically limited in this application.

When N is an integer greater than 0, there is at least one second electronic device in the ovarian health evaluation system. A part or all of the N second electronic devices may be used as data collection devices, configured to collect user vital sign data. A part or all of the N second electronic devices may be used as service provision devices, configured to provide services. The services may include a music playing service, a temperature adjustment service, a video playing service, and the like.

The first electronic device may obtain the user vital sign data (which may also be referred to as a physiological parameter of the user) to evaluate an ovarian function of the user. The user vital sign data may include a skin temperature, a heart rate, a respiratory rate, heart rate variability, a sleep status indicator, a blood pressure, a body fat, an emotion, and the like of the user within a preset time period. A part or all of the user vital sign data may be obtained through a sensor on the first electronic device. Optionally, a part of the user vital sign data may be obtained through a sensor on the N second electronic devices. Optionally, the first electronic device may further obtain data input by the user, and evaluate the ovarian function of the user based on the user vital sign data and the data input by the user. After determining an ovarian health evaluation result of the user (for example, an ovarian health degree of the user), the first electronic device may output the ovarian health evaluation result and feed back the ovarian health evaluation result to the user, so that the user can detect an abnormal ovarian function in a timely manner.

Optionally, the first electronic device may further provide an intelligent solution for the user based on the ovarian health evaluation result, and may further provide a targeted solution while detecting the abnormal ovarian function of the user in a timely manner, to alleviate related symptoms in an ovarian aging process, or help delay an ovarian aging speed, and improve user experience.

For example, the intelligent solution may include performing knowledge push on the first electronic device and/or the second electronic device, for example, pushing knowledge related to diet collocation, scientific exercise, healthy life, and sleep improvement, to help the user build a healthy life, diet, and exercise status. When the ovarian health evaluation system includes the N second electronic devices, the intelligent solution may further include smart home collaboration. For example, when predicting that the user has a hot flash, the first electronic device intelligently adjusts an air conditioner temperature and indoor humidity, to alleviate symptoms of the hot flash of the user; and when sleep quality of the user is poor, the first electronic device plays soothing music through a smart speaker before sleeping or during sleeping, to improve the sleep quality. Further descriptions will be provided subsequently, and details are not described herein.

It may be understood that the ovarian health evaluation system in FIG. 2 is merely some example implementations provided in this embodiment of the present invention, and the ovarian health evaluation system in this embodiment of the present invention includes but is not limited to the foregoing implementations.

The following describes user interfaces provided in embodiments of this application.

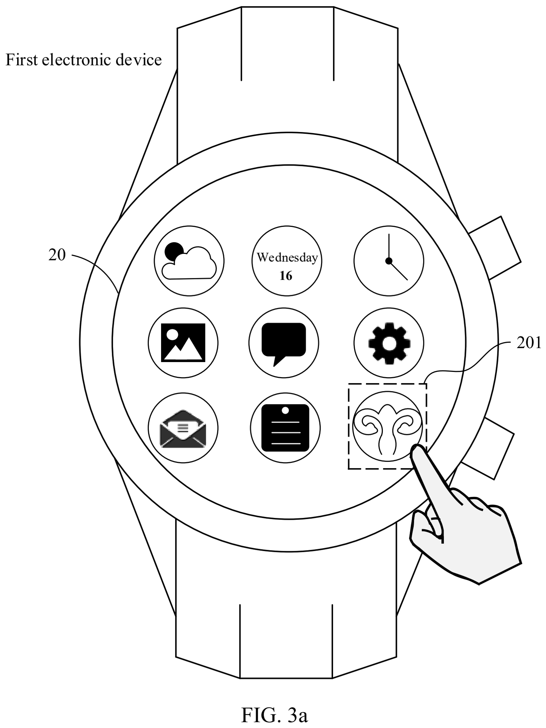

FIG. 3a to FIG. 3i are diagrams of a group of user interfaces displayed on a first electronic device according to an embodiment of this application. The following uses an example in which the first electronic device is a smartwatch for description.

As shown in FIG. 3a, icons of one or more application installed on the first electronic device are displayed on a user interface 20. For example, an ovarian health application control 201, weather, calendar, clock, album, messages, settings, and email are displayed on the user interface 20. Motion time record, heart rate test, temperature test, sleep monitoring, respiratory rate monitoring, and the like that are not displayed in FIG. 3a may be further displayed on the user interface 20.

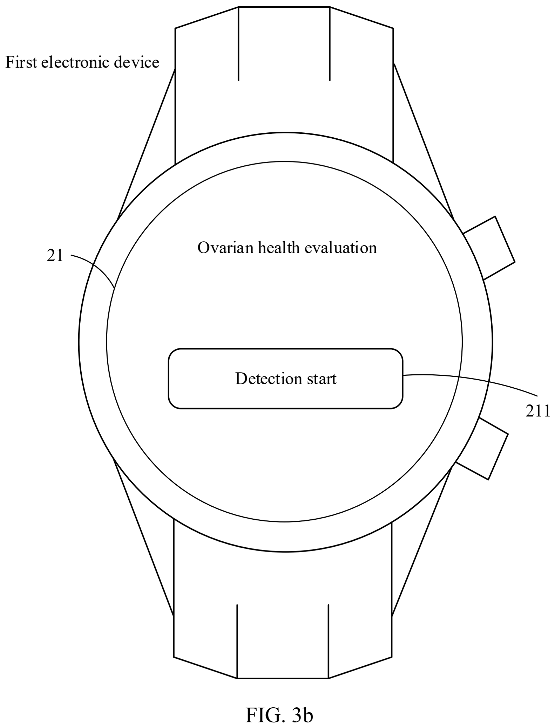

As shown in FIG. 3a, the first electronic device may receive a touch operation of a user on an icon of the ovarian health application control 201 of the user interface 20 (namely, an operation of starting an ovarian health evaluation application), start the ovarian health evaluation application, and display a user interface 21 shown in FIG. 3b on a display of the first electronic device. The user interface 21 may include a detection start control 211.

As shown in FIG. 3b, the first electronic device may receive a touch operation performed by the user on the detection start control 211 of the user interface 21, and then the first electronic device may monitor various physical indicators of the user. The first electronic device may further collaborate with N second electronic devices to monitor the various physical indicators of the user together. In this way, the first electronic device may obtain user vital sign data to evaluate an ovarian function of the user. The user vital sign data may include but is not limited to data such as a skin temperature, a heart rate, a respiratory rate, heart rate variability, a sleep status indicator, a blood pressure, a body fat, and an emotion of the user within a preset time period.

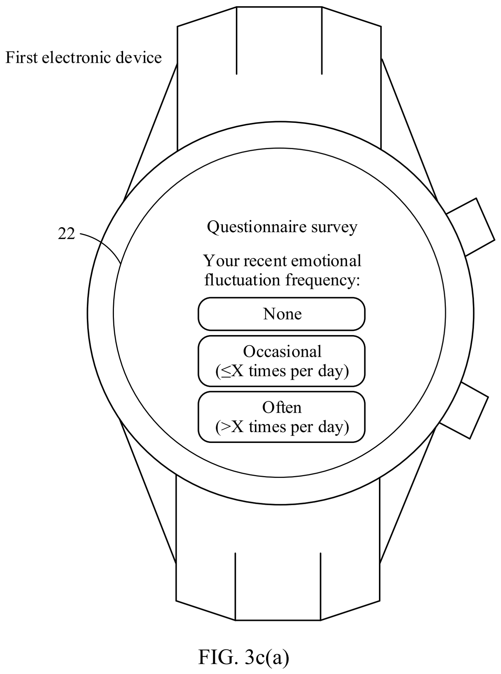

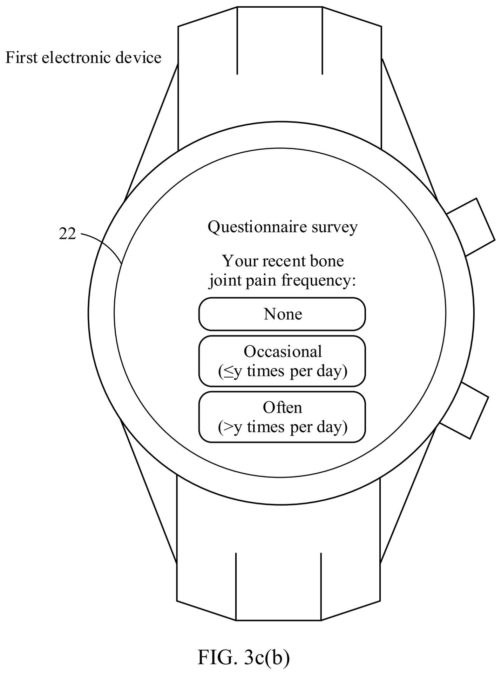

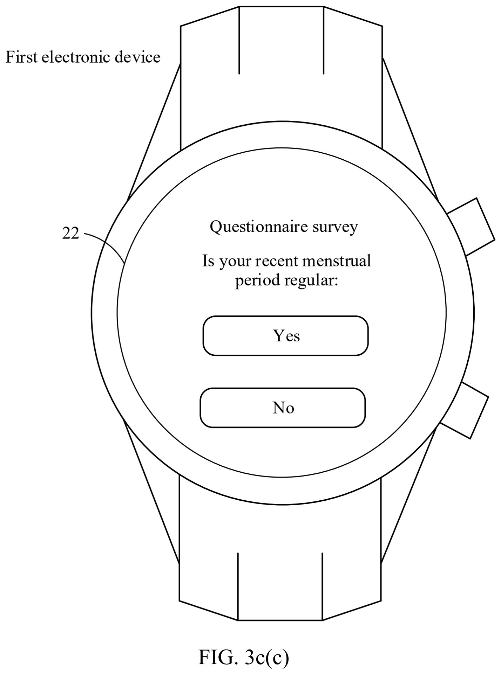

As shown in FIG. 3c(a) to FIG. 3c(c), a user interface 22 may include questionnaire survey content and one or more preset controls. The questionnaire survey content may include one or more questions. When the questionnaire survey content may include a plurality of questions, the plurality of questions may be displayed on the user interface 22 one by one.

As shown in FIG. 3c(a), the question of the questionnaire survey, for example, "Your recent emotional fluctuation frequency", and three preset controls may be displayed on the user interface 22. The three preset controls may respectively correspond to "None", "Occasional (≤x times per day)", and "Often (>x times per day)". Herein, x is a preset threshold. The first electronic device receives a touch operation performed by the user on one of the three preset controls of the user interface 22, records, as a questionnaire survey result input by the user, information corresponding to the preset control selected by the user, and may display a next questionnaire survey question on the user interface 22.

As shown in FIG. 3c(b), the question of the questionnaire survey, for example, "Your recent bone joint pain frequency", and three preset controls may be displayed on the user interface 22. The three preset controls respectively correspond to "None", "Occasional (≤y times per day)", and "Often (>y times per day)". Herein, y is a preset threshold. The first electronic device receives a touch operation performed by the user on one of the three preset controls of the user interface 22, records, as a questionnaire survey result input by the user, information corresponding to the preset control selected by the user, and may display a next questionnaire survey question on the user interface 22.

As shown in FIG. 3c(c), the question of the questionnaire survey, for example, "Is your recent menstrual period regular", and two preset controls may be displayed on the user interface 22. The two preset controls respectively correspond to "Yes" and "No". The first electronic device receives a touch operation performed by the user on one of the two preset controls of the user interface 22, records, as a questionnaire survey result input by the user, information corresponding to the preset control selected by the user, and displays a next questionnaire survey question on the user interface 22. If there is no new questionnaire survey question, the questionnaire survey may be ended. It may be understood that a quantity of preset controls of the user interface 22 may be preset based on a questionnaire question. This is not specifically limited in this application.

In some embodiments, the first electronic device may evaluate the ovarian function of the user based on the obtained user vital sign data and the questionnaire survey result input by the user, so that whether the ovarian function of the user is abnormal can be found more accurately and in a more timely manner. How to evaluate the ovarian function of the user may be further described subsequently, and details are not described herein.

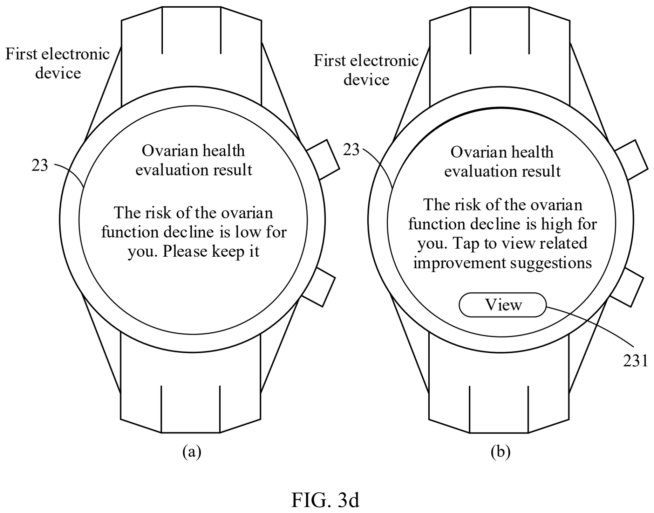

After the preset time period, the first electronic device may receive a touch operation performed by the user on the icon of the ovarian health application control 201 of the user interface 20 (namely, the operation of starting an ovarian health evaluation application), re-start the ovarian health evaluation application, and display a user interface 23 shown in FIG. 3d on the display of the first electronic device. The user interface 23 may include an ovarian health evaluation result.

As shown in (a) in FIG. 3d, after evaluating the ovarian function of the user based on the user vital sign data, if the evaluation result is that a risk of an ovarian decline of the user is low, the first electronic device may display prompt information indicating that the risk of the ovarian decline is low on the user interface 23, for example, "The risk of the ovarian function decline is low for you. Please keep it".

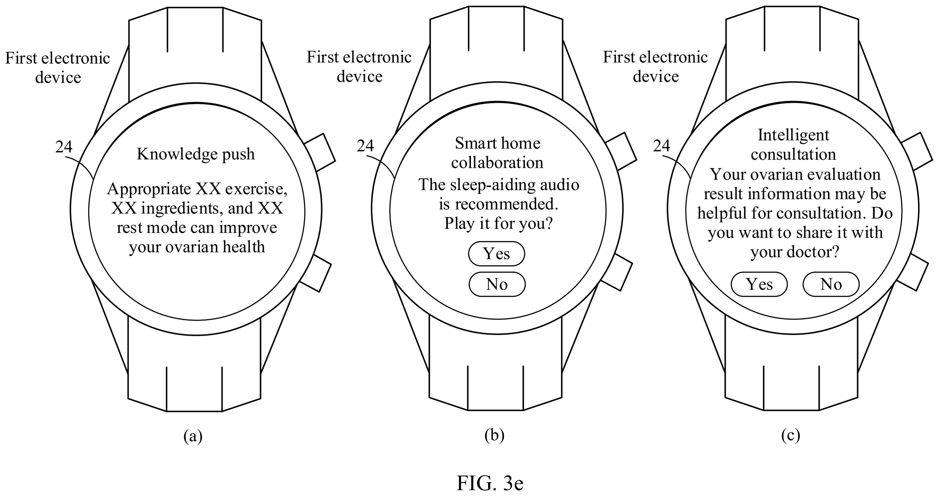

As shown in (b) in FIG. 3d, after evaluating the ovarian function of the user based on the user vital sign data, if the evaluation result is that a risk of an ovarian decline of the user is high, the first electronic device may display prompt information indicating that the risk of the ovarian decline is high on the user interface 23, for example, "The risk of the ovarian function decline is high for you". Optionally, the prompt information may further include "Tap to view related improvement suggestions". The user interface 23 further includes a preset control 231. The first electronic device may receive a touch operation performed by the user on the preset control 231 of the user interface 23, and a user interface 24 shown in FIG. 3e is displayed on the display of the first electronic device.

As shown in (a) in FIG. 3e, the user interface 24 includes knowledge push content, the knowledge push content is determined based on the user ovarian evaluation result, and the knowledge push content may be "Appropriate XX exercise, XX ingredients, and XX rest mode can improve your ovarian health".

As shown in (b) in FIG. 3e, when the first electronic device is connected to the N second electronic devices, and the N second electronic devices include a smart home, for example, a speaker, the user interface 24 may include smart home collaboration information, and the smart home collaboration information may be a prompt indicating whether to collaborate with a smart home. For example, a prompt "The sleep-aiding audio is recommended. Play it for you?" is displayed on the user interface 24. The user interface 24 may further include two preset controls, and the two preset controls respectively correspond to "Yes" and "No". The first electronic device receives a touch operation performed by the user on one of the two preset controls of the user interface 24. If the preset control selected by the user corresponds to "Yes", the first electronic device may indicate the speaker to play the recommended audio. The first electronic device collaborates, based on the user ovarian evaluation result, with the smart home to change a user environment, to improve ovarian health of the user, so as to improve user experience.

In some embodiments, when the first electronic device determines that sleep quality of the user is poor, the first electronic device may automatically play an audio, to improve the user environment, and assist the user in inputting a sleep state, so as to improve user experience.

As shown in (c) in FIG. 3e, the user interface 24 may include intelligent consultation information, and the intelligent consultation information may be a prompt for prompting the user whether the evaluation result needs to be synchronized to a doctor, for example, "Your ovarian evaluation result information may be helpful for consultation. Do you want to share it with your doctor?". The user interface 24 may further include two preset controls, and the two preset controls respectively correspond to "Yes" and "No". The first electronic device receives a touch operation performed by the user on one of the two preset controls of the user interface 24. If the preset control selected by the user corresponds to "Yes", the first electronic device may send the ovarian health evaluation result to a target application. The target application may be a system application and/or a third-party application, and the target application presents the ovarian health evaluation result and/or the related detection data to the doctor with user's consent, to reduce consultation complexity of the user, and improve diagnosis accuracy and efficiency of the doctor, so as to implement an intelligent consultation service and improve user experience.

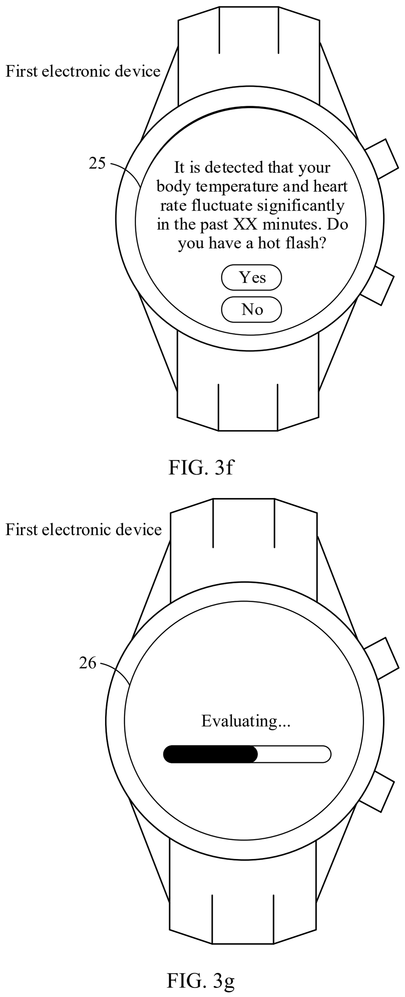

As shown in FIG. 3f, in a process in which the first electronic device detects the user vital sign data, if the first electronic device detects, based on the determined vital sign data, that the user has a hot flash, the first electronic device displays a user interface 25, where the user interface 25 includes a hot flash confirmation prompt, and the hot flash confirmation prompt may be "It is detected that your skin temperature and heart rate fluctuate significantly in the past XX minutes. Do you have a hot flash?". The user interface 25 may further include two preset controls, and the two preset controls respectively correspond to "Yes" and "No". The first electronic device receives a touch operation performed by the user on one of the two preset controls of the user interface 24. If the preset control selected by the user corresponds to "No", the first electronic device may optimize a hot flash detection algorithm based on a hot flash status fed back by the user. It should be noted that, in a process in which the first electronic device evaluates an ovary of the user based on the user vital sign data, a case in which the first electronic device first detects, based on the user vital sign data and the hot flash detection algorithm, that the user has a hot flash may be involved, and further, a case in which the user has a hot flash is used as a feature to evaluate the ovary of the user. This may be described in detail below, and details are not described herein.

As shown in FIG. 3g, before the preset time period, after receiving the operation performed by the user on the ovarian health application control 201 of the user interface 20, the first electronic device may display a user interface 26. Display content of the user interface 26 may include a prompt indicating that detection is being performed, and the display content of the user interface 26 may further include an evaluation progress. In FIG. 3g, the evaluation progress is displayed in a form of progress bar, or the evaluation progress may be displayed in a form of progress ring, number, or the like. This is not specifically limited in this application.

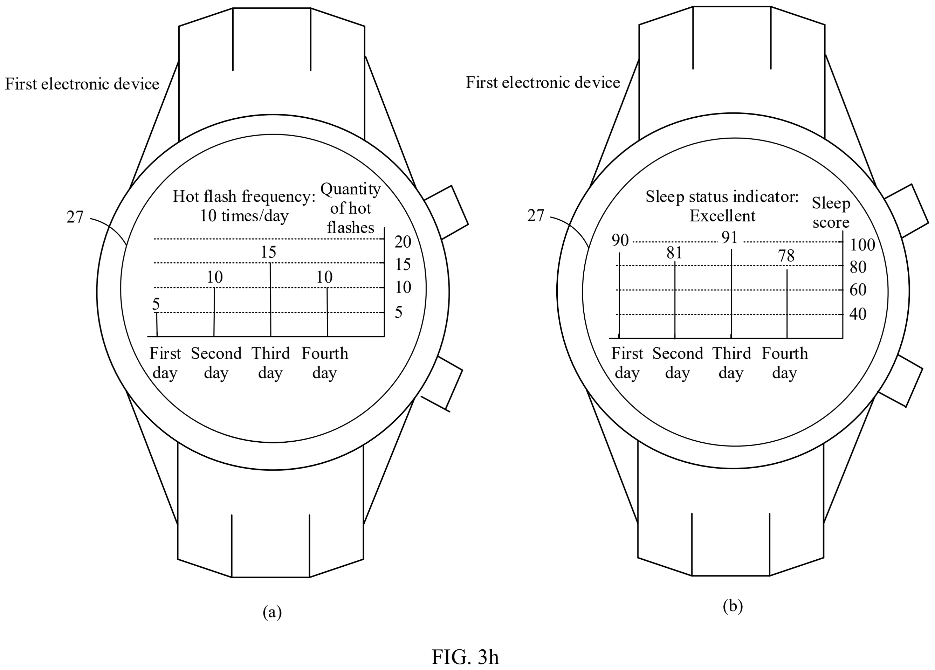

As shown in FIG. 3h, after determining the user ovarian evaluation result, the first electronic device may further display a user interface 27. For example, when receiving a preset operation performed by the user on the user interface 20, the first electronic device may display the user interface 27. The preset operation may include but is not limited to a left sliding operation, a right sliding operation, a mid-air operation, and the like. This is not specifically limited in this application. The user interface 27 may be a symptom information display interface of the user. The user interface 27 may be used to display symptom information of the user. The symptom information may include but is not limited to a hot flash frequency, a sleep status indicator, and the like of the user, so that the user can view information related to the user, to improve user experience.

As shown in (a) in FIG. 3h, the user interface 27 includes the hot flash frequency of the user. For example, the hot flash frequency of the user is 10 times/day. Optionally, the user interface 27 may further include a quantity of times that the user has a hot flash per day within the preset time period. For example, the user has a hot flash five times on a first day, the user has a hot flash 10 times on a second day, the user has a hot flash 15 times on a third day, and the user has a hot flash 10 times on a fourth day.

As shown in (b) in FIG. 3h, the user interface 27 includes the sleep status indicator of the user. For example, the sleep status indicator of the user is excellent. Optionally, the user interface 27 may further include a sleep score of the user per day within the preset time period. For example, the sleep score performed by the user on the first day is 90, the sleep score performed by the user on the second day is 81, the sleep score performed by the user on the third day is 91, and the sleep score performed by the user on the fourth day is 78.

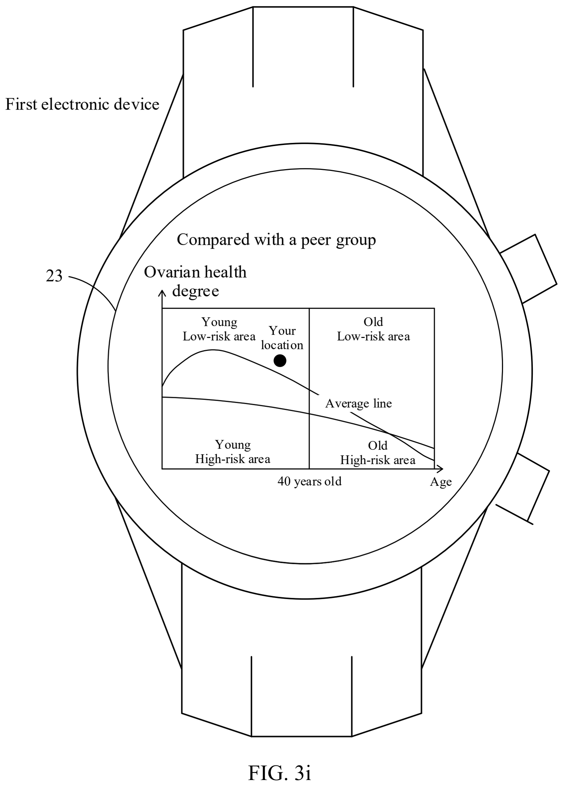

As shown in FIG. 3i, the user interface 23 may further include information compared with a peer group. Optionally, the first electronic device may draw, based on a relationship between an age and an ovarian health degree, a relationship diagram between an age and an ovarian health degree shown in FIG. 3i. Optionally, the first electronic device may further determine, based on the relationship between the age and the ovarian health degree, an ovarian health degree corresponding to each age, to draw an average line shown in FIG. 3i. Optionally, the first electronic device may obtain an age of the user, and then the first electronic device may determine, based on the age of the user and the ovarian health degree, a location of the current ovarian health degree of the user in the relationship diagram between the age and the ovarian health degree, to prompt the user with an ovarian health status of the user compared with the peer group, so as to improve user experience.

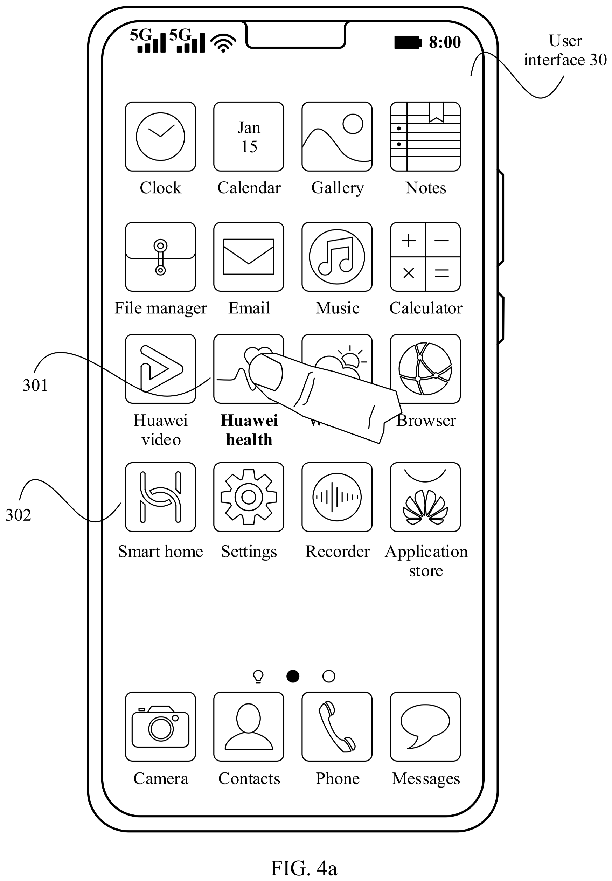

When the first electronic device is connected to the N second electronic devices, FIG. 4a to FIG. 4e(b) are diagrams of a group of user interfaces displayed on the second electronic device according to an embodiment of this application. The following uses an example in which the second electronic device is a smartphone for description.

As shown in FIG. 4a, icons of one or more installed application are displayed on a user interface 30. For example, Huawei health 301, Smart home 302, Clock, Calendar, Notes, File manager, Email, Music, Calculator, Huawei video, Health, Weather, Browser, AI Life, Settings, and Recorder are displayed on the user interface 30.

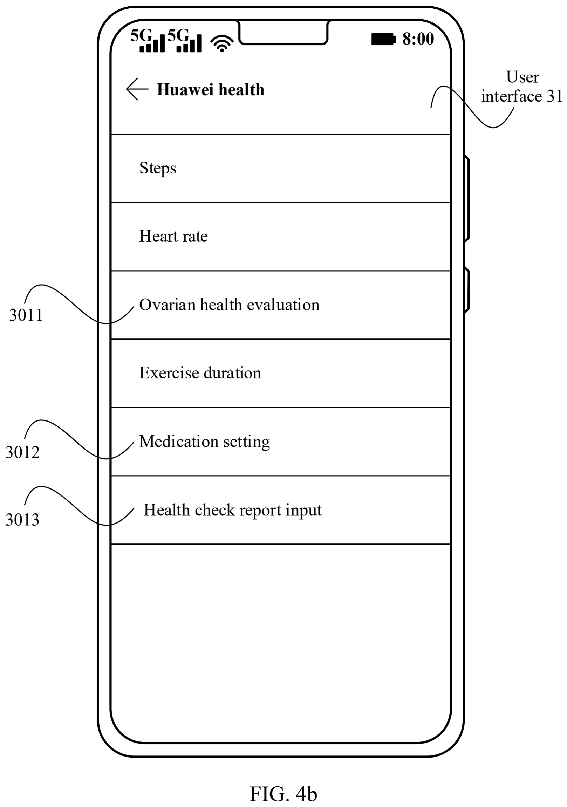

As shown in FIG. 4a, the second electronic device may receive a touch operation performed by the user on an icon of the Huawei health 301 of the user interface 30 (that is, an operation of starting the Huawei health application), start the Huawei health application, and display a user interface 31 shown in FIG. 4b on a display of the second electronic device. The user interface 31 may include an Ovarian health evaluation control 3011, a Medication setting 3012, and a Health check report input 3013.

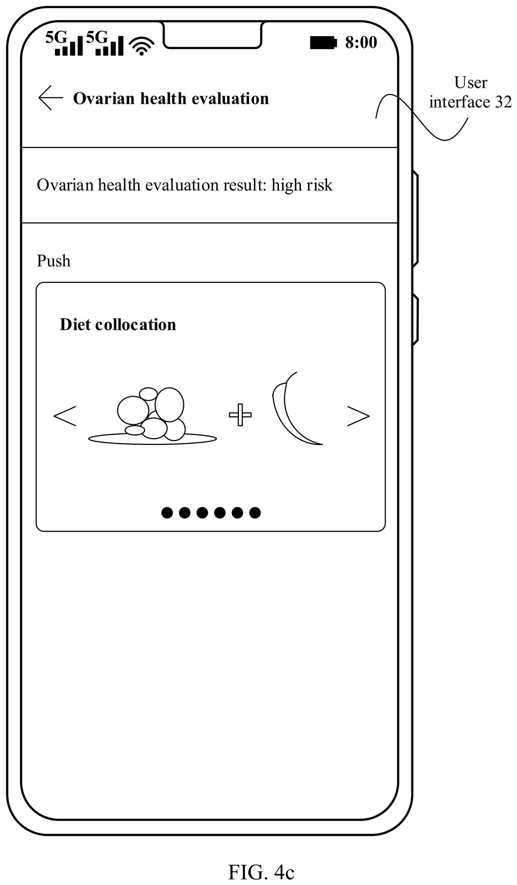

As shown in FIG. 4b, the second electronic device may receive a touch operation performed by the user on the Ovarian health evaluation control 3011 of the user interface 31, and then display a user interface 32 shown in FIG. 4c on the display. The user interface 32 may include ovarian health evaluation result information. Optionally, the user interface 32 may further include knowledge push information determined based on an ovarian health evaluation result.

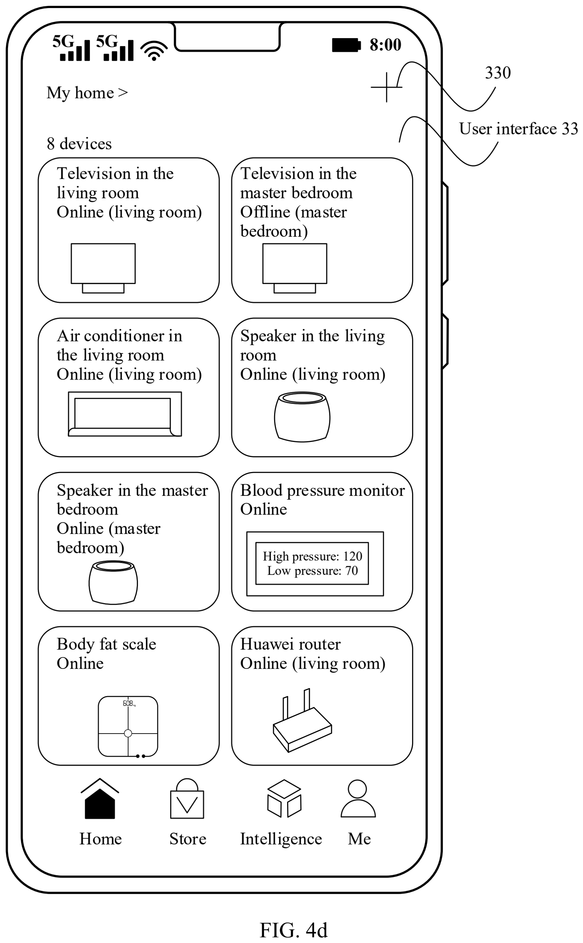

As shown in FIG. 4a, the second electronic device may receive a touch operation performed by the user on an icon of the Smart home 302 of the user interface 30 (that is, an operation of starting the Smart home application), start the Smart home application, and display a user interface 33 shown in FIG. 4d on the display of the second electronic device. The user interface 33 may include connection statuses of smart homes, for example, currently, a television in the living room is online, an air conditioner in the living room is online, a speaker in the living room is online, a speaker in the master bedroom is online, a blood pressure monitor is online, a body fat scale is online, and a Huawei router is online. It should be noted that the N second electronic devices may include a smart terminal (for example, a smartphone) and a smart home. "Online" may be understood as that the smart terminal may establish a connection to the smart home, to implement a whole-house intelligent function. In some embodiments, the smart terminal may be indirectly connected to the smart home, and the smart terminal can manage the home. Specifically, when the first electronic device establishes a connection to the smart terminal, the smart terminal may establish a connection to the smart home, so that the first electronic device may indirectly establish a connection to the smart home. The user interface 33 may further include a preset control 330. The second electronic device may receive a touch operation performed by the user on the preset control 330 of the user interface 33, to establish a new connection to another smart home.

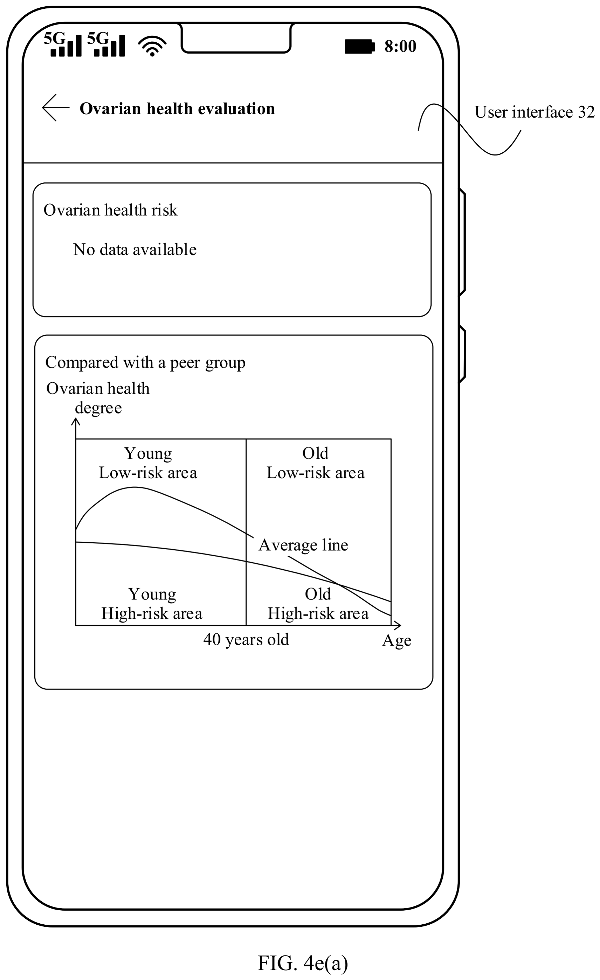

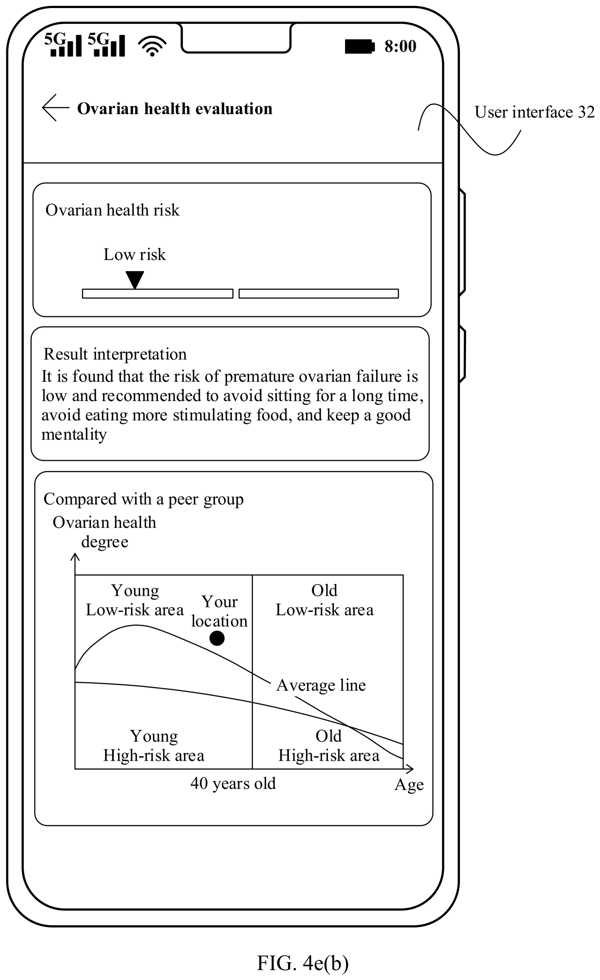

As shown in FIG. 4e(a), before a preset time period, after receiving the operation performed by the user on the Ovarian health evaluation control 3011 of the user interface 31, the second electronic device may display the user interface 32 shown in FIG. 4e(a). The user interface 32 may include prompt information indicating no data available, for example, "No data available about an ovarian health risk". Optionally, the user interface 32 may further include a relationship diagram between an age and an ovarian health degree. Optionally, the second electronic device may draw, based on a relationship between an age and an ovarian health degree, a relationship diagram between an age and an ovarian health degree shown in FIG. 4e(a). Optionally, the second electronic device may further determine, based on the relationship between the age and the ovarian health degree, an ovarian health degree corresponding to each age, to draw an average line shown in FIG. 4e(a).

As shown in FIG. 4e(b), after a preset time period, after receiving the operation performed by the user on the Ovarian health evaluation control 3011 of the user interface 31, the second electronic device may display the user interface 32 shown in FIG. 4e(b). The user interface 32 may include the ovarian health evaluation result of the user, for example, a low ovarian health risk. Optionally, the user interface 32 may further include a relationship diagram between an age and an ovarian health degree. Optionally, the second electronic device may draw, based on a relationship between an age and an ovarian health degree, a relationship diagram between an age and an ovarian health degree shown in FIG. 4e(b). Optionally, the second electronic device may further determine, based on the relationship between the age and the ovarian health degree, an ovarian health degree corresponding to each age, to draw an average line shown in FIG. 4e(b). Optionally, the second electronic device may obtain an age of the user, and then the second electronic device may determine, based on the age of the user and the ovarian health degree, a location of the current ovarian health degree of the user in the relationship diagram between the age and the ovarian health degree, to prompt the user with an ovarian health status of the user compared with the peer group, so as to improve user experience. Optionally, the user interface 32 may further include result interpretation content. For example, the result interpretation content may be "It is found that the risk of premature ovarian failure is low and it is recommended to avoid sitting for a long time, avoid eating more stimulating food, and keep a good mentality".

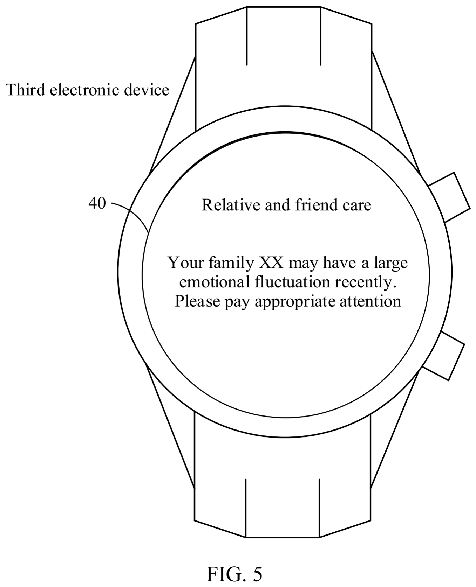

FIG. 5 is a diagram of a user interface displayed on a third electronic device according to an embodiment of this application. The following uses an example in which the third electronic device is a smartwatch for description.

A first electronic device and the third electronic device are bound as a family relationship through a cloud server. After the first electronic device evaluates an ovary of a user based on user vital sign data, the first electronic device may send an ovarian health evaluation result to the cloud server. The cloud server may determine family care prompt information based on the ovarian health evaluation result, and the cloud server may send the family care prompt information to the third electronic device. After receiving the family care prompt information, the third electronic device may display a user interface 40 shown in FIG. 5. The user interface 40 may include the family care prompt information (which may also be referred to as relative and friend care). The relative and friend care may be "Your family XX may have a large emotional fluctuation recently. Please pay appropriate attention".

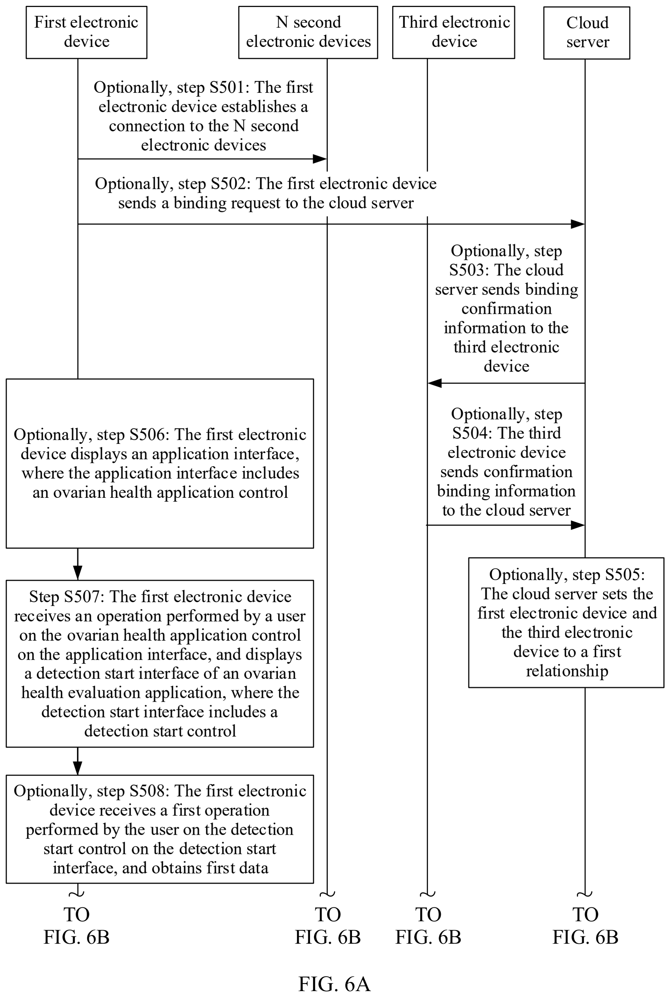

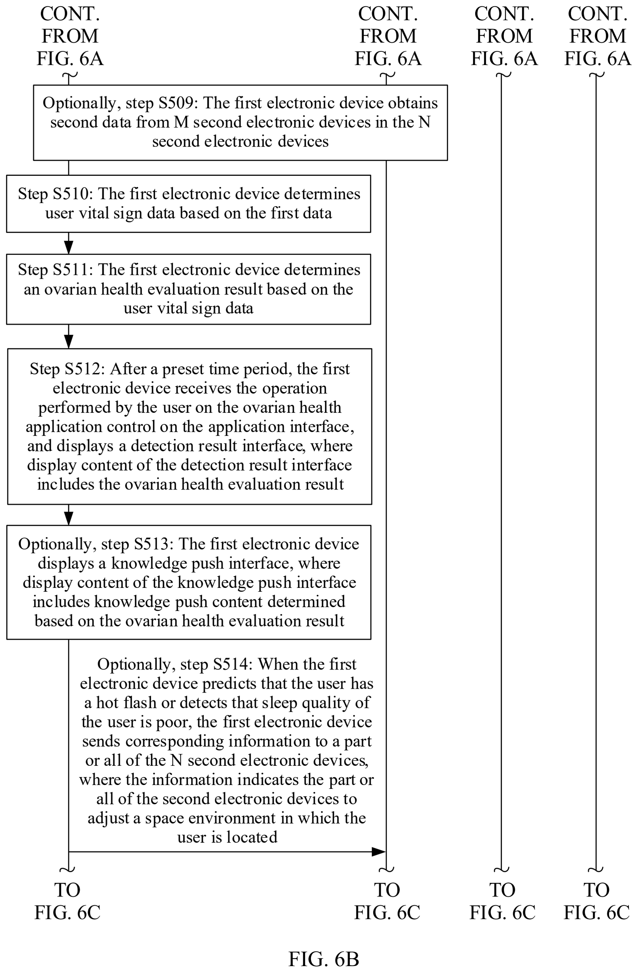

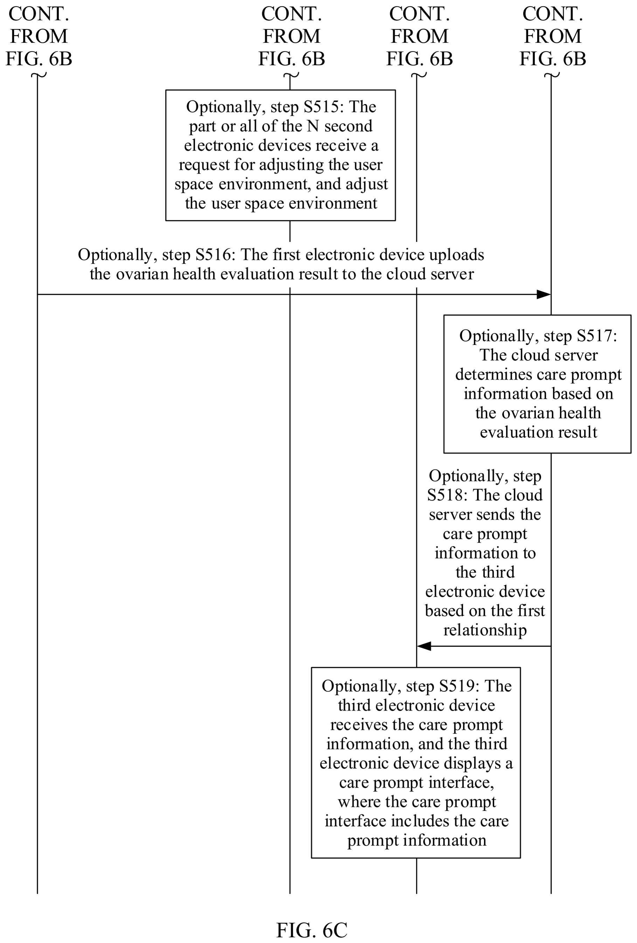

The following describes in detail an ovarian health evaluation method provided in an embodiment of this application with reference to FIG. 6A to FIG. 6C. Details are described below.

Optionally, step S501: A first electronic device establishes a connection to N second electronic devices.

Specifically, the first electronic device may be a device that can be used to measure physiological data of a user, for example, a wearable device. For example, the first electronic device may be a smartwatch, a smart band, a smart ring, or the like. N is an integer greater than 0. The N second electronic devices may include a terminal and/or a smart home. For example, the N second electronic devices may include a smartphone, a smart speaker, a body fat scale, an air conditioner, a blood pressure monitor, and the like. The first electronic device may establish a connection to the N second electronic devices by using a wireless technology, so that the first electronic device can work with the N second electronic devices cooperatively.

It may be understood that the first electronic device may be directly connected to the second electronic device, or the first electronic device may be indirectly connected to the second electronic device. It may be understood that the first electronic device may be indirectly connected to the second electronic device through another device. In this case, the first electronic device may send information to the second electronic device through the another device.

Optionally, step S502: The first electronic device sends a binding request to a cloud server.

Specifically, the first electronic device may establish a first relationship with a third electronic device, and the first electronic device may send the binding request to the cloud server. The binding request may include but is not limited to a request for establishing the first relationship with the third electronic device, that is, the first relationship may be a family relationship, a friend relationship, a relative relationship, or the like. The third electronic device may be an electronic device like a smartphone or a smartwatch. After the first electronic device establishes the first relationship with the third electronic device, and the first electronic device determines an ovarian health evaluation result of the user, the first electronic device may upload the ovarian health evaluation result to the cloud server, the cloud server may determine care prompt information based on the ovarian health evaluation result, and further, the cloud server may send the care prompt information to the third electronic device, so that the first electronic device collaborates with the third electronic device to perform relative and friend care. For example, the first electronic device pushes, to a relative or friend, information that a case such as physical discomfort or an emotional fluctuation may occur on the user, so that the relative or friend makes preparations in advance, provides more care, and avoids an emotional conflict, to reduce user discomfort.

Optionally, step S503: The cloud server sends binding confirmation information to the third electronic device.

Specifically, after receiving the binding request sent by the first electronic device, the cloud server may send the binding confirmation information to the third electronic device, where the binding confirmation information is used to determine whether to establish the first relationship with the first electronic device.

Optionally, step S504: The third electronic device sends confirmation binding information to the cloud server.

Specifically, after the user confirms, on the third electronic device, to establish the first relationship with the first electronic device, the third electronic device may send the confirmation binding information to the cloud server.

Optionally, step S505: The cloud server sets the first electronic device and the third electronic device to the first relationship.

Specifically, the first relationship may include a family relationship, a friend relationship, a relative relationship, and the like.

It should be noted that step S502 to step S504 may be performed at any moment after step S501 and before step S519, or may be performed before step S501. This is not specifically limited in this application.

Optionally, step S506: The first electronic device displays an application interface, where the application interface includes an ovarian health application control.

Specifically, the application interface may be the user interface 20 mentioned above, and the ovarian health application control may be the ovarian health application control 201 of the user interface 20. For detailed descriptions of the user interface 20, refer to the foregoing descriptions of FIG. 3a. Details are not described herein again. The application interface may alternatively be the user interface 30 mentioned above. For detailed descriptions of the user interface 30, refer to the foregoing descriptions of FIG. 4a. Details are not described herein again.

Step S507: The first electronic device receives an operation performed by the user on the ovarian health application control on the application interface, and displays a detection start interface of the ovarian health evaluation application, where the detection start interface includes a detection start control.

Specifically, the detection start interface may be the user interface 21, and the detection start control included in the detection start interface may be the detection start control 211. For detailed descriptions of the user interface 21, refer to the foregoing descriptions of FIG. 3b. Details are not described herein again. The detection start interface may alternatively be an interface displayed after the user performs a touch operation on the Ovarian health evaluation control 3011 of the user interface 31. The interface may include the detection start control.

In some embodiments, the detection start interface may be a first interface, and the detection start control may be a first control.

Step S508: The first electronic device receives a first operation performed by the user on the detection start control on the detection start interface, and obtains first data.

Specifically, the first data may be understood as data detected by the first electronic device. The first data includes heart rate variability data of the user within a preset time period. The first data may further include one or more of heart rate data of the user within the preset time period and respiratory rate data of the user within the preset time period. The first data may further include one or more of skin temperature data of the user within the preset time period, a sleep status indicator of the user within the preset time period, and the like. After receiving the first operation performed by the user on the detection start control on the detection start interface, the first electronic device may obtain the first data. Optionally, after the first electronic device receives the first operation performed by the user on the detection start control on the detection start interface, the first electronic device may monitor a first vital sign indicator of the user within the preset time period. After the preset time period, the first electronic device obtains the first data. A plurality of sensors, such as a temperature sensor, an optical heart rate sensor, a gyro sensor, and an acceleration sensor, may be integrated into the first electronic device. The first vital sign indicator of the user may include heart rate variability, and the first vital sign indicator of the user may further include a heart rate, a skin temperature, a respiratory rate, a sleep score, a quantity of waking-up in a sleep process, and the like. The first operation may be a touch operation, a sliding operation, a mid-air operation, or the like. For example, the user may touch the detection start control 211 on the first electronic device. The first electronic device may receive an operation performed by the user on the detection start control on the detection start interface. The first electronic device may start the temperature sensor to detect the skin temperature of the user. The first electronic device may start the optical heart rate sensor to detect the heart rate, the respiratory rate, the heart rate variability, and the like of the user. The first electronic device may also start the sensors such as the optical heart rate sensor, the acceleration sensor, and the gyro sensor to detect the sleep score, the quantity of waking-up in the sleep process (that is, detect the sleep status indicator), and the like of the user.

In some embodiments, a sleep parameter of the user within the preset time period may include the sleep score, the quantity of waking-up in the sleep process, and the like.

Optionally, step S509: The first electronic device obtains second data from M second electronic devices in the N second electronic devices.

Specifically, N is an integer greater than 0, and M is an integer greater than 0 and less than or equal to N. Optionally, the second data obtained by the first electronic device from the M second electronic devices in the N second electronic devices may be understood as historical data detected by the M second electronic devices in the N second electronic devices. For example, the second data may include historical blood pressure data, historical body fat data, and historical emotion data of the user. In some embodiments, the second data may include blood pressure data in the historical blood pressure data of the user within the preset time period, body fat data in the historical body fat data within the preset time period, and emotion data in the historical emotion data within the preset time period. Optionally, the second data may alternatively be data stored in the M second electronic devices. For example, the M second electronic devices include a smartphone, and the second data may alternatively be historical heart rate data stored in the smartphone. Optionally, the data stored in the M second electronic devices may be data determined by a part or all of the N second electronic devices by monitoring the user vital sign. Optionally, the data stored in the M second electronic devices may alternatively be data determined by a device other than the N second electronic devices by detecting the user vital sign.

In some embodiments, the first electronic device may send a detection request to the N second electronic devices when or after receiving the operation performed by the user on the detection start control on the detection start interface; and the first electronic device obtains the second data from the M second electronic devices in the N second electronic devices. The detection request may include requesting the second electronic device to detect a physical indicator of the user. For example, when the N second electronic devices include a blood pressure monitor, a body fat scale, and a smart screen, the detection request sent by the first electronic device to the blood pressure monitor may include requesting to detect a blood pressure of the user; the detection request sent by the first electronic device to the body fat scale may include requesting to detect a body fat of the user; and the detection request sent by the first electronic device to the smart screen may include requesting to detect a user emotion. The second data may be understood as data detected by the M second electronic devices. The second data may include blood pressure data of the user detected by the blood pressure monitor within the preset time period. The second data may include body fat data of the user detected by the body fat scale within the preset time period. The second data may include user emotion data detected by the smart screen within the preset time period.

In some embodiments, step S509 may be performed before step S508, or step S509 may be performed after step S508. This is not specifically limited in this application.

Step S510: The first electronic device determines user vital sign data based on the first data.

Specifically, the user vital sign data may include a part or all of the first data. For example, the user vital sign data may include one or more of the following: heart rate data of the user within the preset time period, respiratory rate data of the user within the preset time period, and heart rate variability data of the user within the preset time period. The first data may further include skin temperature data of the user within the preset time period, a sleep status indicator of the user within the preset time period, and the like. The user vital sign data may be used as a basis for evaluating ovarian health of the user, so that an ovarian health status of the user can be determined.

In some embodiments, the first electronic device may obtain the second data from the M second electronic devices in the N second electronic devices, for example, step S509. Further, the first electronic device determines the user vital sign data based on the first data and the second data.

Specifically, the user vital sign data may include the first data and the second data. For example, the user vital sign data may include but is not limited to the skin temperature data of the user within the preset time period, the heart rate data of the user within the preset time period, the respiratory rate data of the user within the preset time period, the heart rate variability data of the user within the preset time period, the sleep status indicator of the user within the preset time period, the blood pressure data of the user within the preset time period, the body fat data of the user within the preset time period, and the emotion data of the user within the preset time period. The user vital sign data may be used as a basis for evaluating ovarian health of the user, that is, input data of an ovarian function evaluation model may be determined based on the user vital sign data. In this application, an advantage of multi-device collaboration can be fully used, and a plurality of intelligent devices can be collaborated to provide more comprehensive monitoring of various body indicators, to implement multi-dimensional and comprehensive ovarian health evaluation, so as to improve accuracy of the ovarian health evaluation result.

In some embodiments, the first electronic device may further obtain user information, and determine the ovarian health evaluation result based on the user information and the user vital sign data. The user information includes one or more of medication, drinking, a disease history, and a sleep environment temperature of the user. It may be understood that the user information may include but is not limited to information such as the medication, the drinking, the disease history, and the sleep environment temperature of the user. This is not limited in this embodiment of this application.

In a possible implementation, the first electronic device may preprocess the user vital sign data based on the user information, to determine the preprocessed user vital sign data, and may further evaluate the ovarian health of the user based on the preprocessed user vital sign data. In this embodiment of this application, considering that a part of the obtained user vital sign data may have an abnormal fluctuation, if the data is directly used, accuracy of subsequent feature extraction may be reduced, affecting accuracy of ovarian function evaluation. Therefore, the first electronic device may perform data denoising and interference removal operations on the user vital sign data based on the user information, to effectively improve accuracy of feature extraction, so as to improve accuracy of ovarian health evaluation.

In some embodiments, the foregoing preprocessing may include: The first electronic device determines, based on the user information, whether the medication, the drinking, the disease, and the environment temperature of the user affect the vital sign indicator in the user vital sign data. If feature data is abnormal in this case, abnormal data may be removed, so that the affected feature data may not participate in analysis.

For example, based on a sleep detection model, if the first electronic device detects that the user wakes up at night, the first electronic device filters a vital sign parameter that fluctuates greatly in a wake-up period of the user, for example, filters heart rate data in the wake-up period of the user, and filters respiratory rate data in the wake-up period of the user.

For another example, with reference to emotion monitoring, if the first electronic device identifies emotions such as excessive excitement, heavy pressure, panic, or depression, the first electronic device considers filtering vital sign data that may fluctuate greatly in the abnormal time period, for example, heart rate data in the abnormal time period and respiratory rate data in the abnormal time period.

In another possible implementation, the first electronic device may use the user information and the user vital sign data as the input data for the ovarian health evaluation, to determine the ovarian health evaluation result, so as to implement multi-dimensional and comprehensive ovarian health evaluation, and improve accuracy of the ovarian health evaluation result.

Step S511: The first electronic device determines the ovarian health evaluation result based on the user vital sign data.

Specifically, the first electronic device may determine the ovarian health evaluation result based on the ovarian function evaluation model and the user vital sign data. The ovarian function evaluation model may be a neural network algorithm, a weighted algorithm, or the like. The ovarian function evaluation model is not limited in this embodiment of this application. The ovarian function evaluation model may extract a feature from the user vital sign data, or analyze the user vital sign data, to evaluate the ovarian health of the user.

In some embodiments, the first electronic device may use the user vital sign data as the input data of the ovarian health evaluation model, and the ovarian evaluation model may directly analyze and process the user vital sign data, to determine the ovarian health status of the user.

In some embodiments, the first electronic device determines target data based on the user vital sign data; and the first electronic device determines the ovarian health evaluation result based on the target data and the ovarian function evaluation model.

Specifically, the target data may be understood as the input data that is input to the ovarian function evaluation model and that is obtained by processing the user vital sign data.

In some embodiments, the target data may be score values corresponding to all vital sign indicators after scores are given to the plurality of vital sign indicators included in the user vital sign data. Specifically, the first electronic device may score the plurality of vital sign indicators included in the user vital sign data. For example, if the user vital sign data includes the heart rate data of the user within the preset time period, the respiratory rate data of the user within the preset time period, the heart rate variability data of the user within the preset time period, the skin temperature data of the user within the preset time period, the sleep status indicator of the user within the preset time period, the blood pressure data of the user within the preset time period, the body fat data of the user within the preset time period, and the emotion data of the user within the preset time period, the first electronic device may score all the vital sign indicators included in the user vital sign data, to determine the target data. For example, the first electronic device may record a score of the heart rate data within the preset time period as n1, the first electronic device may record a score of the respiratory rate data within the preset time period as n2, the first electronic device may record a score of the heart rate variability data within the preset time period as n3, the first electronic device may record a score of the skin temperature data within the preset time period as n4, the first electronic device may record a score of the sleep status indicator within the preset time period as n5, the first electronic device may record a score of the blood pressure data within the preset time period as n6, the first electronic device may record a score of the body fat data within the preset time period as n7, and the first electronic device may record a score of the emotion data within the preset time period as n8. Herein, n1, n2, n3, n4, n5, n6, n7, and n8 may be used as the target data. Then, the first electronic device may use scores of all the vital sign indicators for calculation according to Formula 1, and multiply the scores of all the vital sign indicators by respective corresponding weights, and then perform summation on results. The first electronic device may determine an ovarian health score of the user. It should be noted that Formula 1 may be the ovarian health evaluation model provided in this embodiment of this application. Formula 1 is merely an implementation provided in this embodiment of this application, and does not specifically limit the ovarian evaluation model in this application. Further, the first electronic device may preset a mapping relationship between an ovarian health score and an ovarian health degree. For example, an ovarian health degree corresponding to a first score interval is a low risk, and an ovarian health degree corresponding to a second score interval is a high risk. After determining the ovarian health score of the user, the first electronic device may determine a current ovarian health degree of the user based on the preset mapping relationship, to implement multi-dimensional and comprehensive ovarian health evaluation, so as to improve accuracy of the ovarian health evaluation result.

$$F = \sum_{i=1}^{i=m} \alpha_i * n_i \quad (\text{Formula } 1)$$

Herein, F may represent the ovarian health score of the user, $\alpha_i$ may represent a weight corresponding to the vital sign indicator, and $n_i$ may represent a score of the vital sign indicator.

In some embodiments, the target data may be symptom information. For example, the user vital sign data includes the heart rate data of the user within the preset time period, the respiratory rate data of the user within the preset time period, the heart rate variability data of the user within the preset time period, the skin temperature data of the user within the preset time period, and the sleep status indicator of the user within the preset time period. The first electronic device may determine a hot flash frequency of the user and evaluate an autonomic nerve function level based on all the vital sign indicators in the user vital sign data. The first electronic device may further determine physiological data of a sleep disorder of the user based on all the vital sign indicators in the user vital sign data. The symptom information may include one or more of a user flash hot status (which may also be referred to as the flash hot frequency), the user autonomic nerve function level, and a user sleep disorder status.

In some embodiments, the target data may be a score value corresponding to a score of a user symptom indicator. Specifically, the first electronic device may first determine the user symptom indicator (which may be referred to as the symptom information) based on the plurality of vital sign indicators included in the user vital sign data. For example, the user vital sign data includes the heart rate data of the user within the preset time period, the respiratory rate data of the user within the preset time period, the heart rate variability data of the user within the preset time period, the skin temperature data of the user within the preset time period, and the sleep status indicator of the user within the preset time period. The first electronic device may determine a hot flash frequency of the user and evaluate an autonomic nerve function level based on all the vital sign indicators in the user vital sign data. The first electronic device may further determine physiological data of a sleep disorder of the user based on all the vital sign indicators in the user vital sign data. The user symptom indicator may include one or more of a user flash hot status (which may also be referred to as the flash hot frequency), the user autonomic nerve function level, and a user sleep disorder status. The user symptom indicator may further include another symptom indicator. This is not specifically limited in this application. Further, the first electronic device may score all the indicators included in the user symptom indicator, to determine the target data. For example, the first electronic device may record a score of the user hot flash status (for example, the hot flash frequency) as N1, the first electronic device may record a score of the user autonomic nerve function level as N2, and the first electronic device may record a score of the user sleep disorder status (for example, an insomnia level) as N3. Herein, N1, N2, and N3 may be used as the target data. Then, the first electronic device may use scores of all the symptom indicators (which may be used as target input data) for calculation according to Formula 2, and multiply the scores of all the symptom indicators by respective corresponding weights, and then perform summation on results, to determine the ovarian health score of the user. It should be noted that Formula 2 may be the ovarian health evaluation model provided in this embodiment of this application. Formula 2 is merely an implementation provided in this embodiment of this application, and does not specifically limit the ovarian evaluation model in this application. Further, after determining the ovarian health score of the user, the first electronic device may determine a current ovarian health degree of the user based on the preset mapping relationship, to implement multi-dimensional and comprehensive ovarian health evaluation, so as to improve accuracy of the ovarian health evaluation result. In this embodiment of this application, original input data is converted into symptoms in different dimensions caused by an ovarian function decline, and a feature that can be directly used for ovarian function evaluation is determined, so that the ovarian health of the user can be evaluated more accurately.

$$ F = \sum_{i=1}^{i=m} \partial_i * N_i \quad (\text{Formula 2}) $$

Herein, F may represent the ovarian health score of the user, $\partial_i$ may represent a weight corresponding to the symptom indicator, and $N_i$ may represent a score of the symptom indicator.

It should be noted that the ovarian health degree may be a low risk or a high risk of the ovarian function decline. Optionally, the ovarian health degree may be further distinguished into a medium risk, a high risk, and the like based on severity.

In some embodiments, the first electronic device may analyze and process the heart rate data of the user within the preset time period and the respiratory rate data of the user within the preset time period based on the hot flash detection algorithm, and may determine the hot flash status of the user, where the hot flash status may include a hot flash frequency. The first electronic device may alternatively determine the hot flash status of the user based on the heart rate data of the user within the preset time period, the respiratory rate data of the user within the preset time period, and the skin temperature data of the user within the preset time period. For example, a weight may be preset for each user vital sign indicator, and then a score may be given to the user vital sign indicator, so that a score of each user vital sign indicator may be multiplied by a preset weight and then the result is added to another result, to determine the hot flash status of the user.

In some embodiments, the first electronic device may obtain one or more of the heart rate data, the respiratory rate data, and the skin temperature data; display a fourth interface when one or more of the heart rate data, the respiratory rate data, and the skin temperature data meet a first condition, where the fourth interface includes a third control and a fourth control, the third control is used by the user to confirm that a hot flash currently occurs, and the fourth control is used by the user to confirm that no hot flash currently occurs; and receive an operation performed by the user on the third control or the fourth control.

Specifically, when one or more of the heart rate data, the respiratory rate data, and the skin temperature data meet the first condition, the first electronic device may determine that the user has a hot flash, so that the first electronic device may display a hot flash confirmation interface, namely, the fourth interface. The hot flash confirmation interface includes two preset controls: the third control and the fourth control. The third control may be used by the user to confirm that a hot flash currently occurs, and the fourth control is used by the user to confirm that no hot flash currently occurs. The first electronic device receives an input operation performed by the user on the hot flash confirmation interface. The hot flash confirmation interface may be the user interface 25 in FIG. 3f. For detailed descriptions of the user interface 25, refer to the foregoing descriptions of FIG. 3f. Details are not described herein again.

In this embodiment of this application, when a hot flash is detected, the first electronic device may prompt the user to confirm whether the hot flash occurs, and may further adjust, based on user feedback, a parameter involved in the first condition, for example, adjust the first preset threshold in the first condition based on user feedback, to improve accuracy of detecting, by the first electronic device, whether the user has a hot flash, so as to improve accuracy of ovarian function evaluation and improve individual interaction experience of the user.

In some embodiments, the first condition is that a data change amplitude of one or more of the heart rate data, the respiratory rate data, and the skin temperature data in a first time interval within the preset time period is greater than or equal to the first preset threshold.

Specifically, the first time interval is a time interval within the preset time period, for example, 5 minutes. When it is detected that one or more of the heart rate data, the respiratory rate data, and the skin temperature data of the user fluctuates greatly in a short time, it may be determined that the user has a hot flash.

In some embodiments, the first electronic device may determine the user autonomic nerve function level based on the heart rate variability data of the user within the preset time period.

In some embodiments, the first electronic device may determine the user sleep disorder status (for example, the insomnia level) based on the sleep status indicator (for example, the sleep score and the quantity of waking-up) of the user within the preset time period.

In some embodiments, the user vital sign data may further include the questionnaire survey data. Specifically, the first electronic device may further display the user questionnaire survey interface, and the user may input the questionnaire survey result on the user questionnaire survey interface. The user questionnaire survey interface may be the user interface 22. For detailed descriptions of the user interface 22, refer to the foregoing descriptions of FIG. 3c(a) to FIG. 3c(c). Details are not described herein again. The user questionnaire survey interface may be a third interface. The first electronic device may receive an input operation performed by the user on the user questionnaire survey interface, and determine the ovarian health evaluation result based on the ovarian function evaluation model and the user vital sign data. Questionnaire survey content includes but is not limited to an osteoporosis symptom, an emotional status, and the like. In this embodiment of this application, the first electronic device may collect data of the user in more dimensions with reference to the questionnaire survey, including but not limited to the osteoporosis symptom, the emotional status, and the like, and the data may be jointly used as the input data of the ovarian health evaluation model, to implement multi-dimensional and comprehensive ovarian health evaluation, so as to improve accuracy of the ovarian health evaluation result. In a possible implementation, the questionnaire survey data includes the questionnaire survey result of the user. In another possible implementation, the first electronic device may score the questionnaire survey result input by the user, and the questionnaire survey data may include a score of the questionnaire survey result.

In some embodiments, the first electronic device may obtain user health check data, where the user health check data may include but is not limited to an anti-mullerian hormone (Anti-Mullerian hormone, AMH) test result and a follicle-stimulating hormone (Follicle-stimulating hormone, FSH) test result. The first electronic device may optimize the ovarian function evaluation model based on the user health check data, for example, a parameter threshold in the ovarian function evaluation model. In this embodiment of this application, the first electronic device obtains corresponding hormone level information of the user, and uses an ovarian health degree corresponding to the hormone level as a golden standard of a current ovarian health degree of the user, so that the parameter threshold in the ovarian function evaluation model can be optimized, a model baseline can be re-established, and the like, to improve accuracy of subsequent detection.

Step S512: After the preset time period, the first electronic device receives the operation performed by the user on the ovarian health application control on the application interface, and displays a detection result interface, where display content of the detection result interface includes the ovarian health evaluation result.

Specifically, after the preset time period, when the user re-starts the ovarian health evaluation application, the ovarian health evaluation result may be displayed on the first electronic device, and the detection result interface may be the user interface 23 mentioned above. For detailed descriptions of the user interface 23, refer to the foregoing descriptions of FIG. 3d. Details are not described herein again. The detection result interface may be a second interface. In this embodiment of this application, the ovarian health evaluation result of the user is displayed on the first electronic device, so that an abnormal ovarian function of the user can be detected in a timely manner. If a symptom of abnormal ovarian aging can be detected in a timely manner, a related symptom caused by ovarian aging can be improved.

In some embodiments, before the preset time period, the first electronic device receives the operation performed by the user on the ovarian health application control on the application interface, and may display a detecting interface. Display content on the detecting interface may include an evaluation progress, which may be displayed in a form of progress bar. The detecting interface may be the user interface 26. For detailed descriptions of the user interface 26, refer to the foregoing descriptions of FIG. 3g. Details are not described herein again.

Optionally, step S513: The first electronic device displays a knowledge push interface, where display content of the knowledge push interface includes knowledge push content determined based on the ovarian health evaluation result.

Specifically, the knowledge push interface may be the user interface 24 mentioned above. For detailed descriptions of the user interface 24, refer to the foregoing descriptions of (a) in FIG. 3d. Details are not described herein again. In this embodiment of this application, knowledge push may be performed on the first electronic device for the user with a low risk of an ovarian decline or the user with a high risk of the ovarian decline, for example, knowledge related to diet collocation, scientific exercise, healthy life, and sleep improvement is pushed, to help the user build a healthy lifestyle, diet, and exercise routine, and provide a targeted solution when the abnormal ovarian function of the user is detected in a timely manner, so as to delay an ovarian aging speed.

In some embodiments, the first electronic device may display the knowledge push content on the leftmost screen of the first electronic device.

In some embodiments, the first electronic device may synchronize the ovarian health evaluation result to a part or all of the N second electronic devices, and display an ovarian health evaluation result interface on the second electronic device. The ovarian health evaluation result interface may include the ovarian health evaluation result. The ovarian health evaluation result interface may be the user interface 32 mentioned above. For detailed descriptions of the user interface 32, refer to the foregoing descriptions of FIG. 4c. Details are not described herein again.

Optionally, step S514: When the first electronic device predicts that the user has a hot flash or detects that the sleep quality of the user is poor, the first electronic device sends corresponding information to a part or all of the N second electronic devices, where the information indicates the part or all of the second electronic devices to adjust a space environment in which the user is located.

Specifically, the first electronic device may further collaborate with the smart home to adjust the user space environment, for example, intelligently adjust an air conditioner temperature and indoor humidity when it is predicted that the hot flash occurs, to alleviate a hot flash symptom of the user. If the sleep quality is poor, soothing music is played before sleeping/during sleeping, to improve the sleep quality.

In some embodiments, the first data further includes the sleep parameter. The N second electronic devices include the smart speaker within the preset time period. Whether the sleep quality of the user is less than a second preset threshold is determined based on the sleep parameter. If the sleep quality of the user is less than the second preset threshold, first information is sent to the smart speaker. The first information indicates the smart speaker to play an audio. Specifically, the sleep quality of the user may be determined based on the sleep score and/or the quantity of waking-up of the user in the sleep parameter (which may also be referred to as the sleep status indicator).

Optionally, the first electronic device may recommend a plurality of audios to the user, and the user may select or preset, based on a personal preference, an audio that needs to be played.

In some embodiments, the N second electronic devices include a smart air conditioner. A target moment at which the user has a hot flash is predicted within the preset time period, and second information is sent to the smart air conditioner before the target moment, where the second information indicates the smart air conditioner to adjust a current temperature.

Optionally, the first electronic device may provide a temperature adjustment option of the smart air conditioner for the user, and the user may select or preset an adjustment range of the air conditioner temperature.

In some embodiments, the first electronic device may predict, based on the user vital sign data, a time at which the user has a hot flash.

Optionally, step S515: The part or all of the N second electronic devices receive a request for adjusting the user space environment, and adjust the user space environment.

Optionally, step S516: The first electronic device uploads the ovarian health evaluation result to the cloud server.

In some embodiments, the first electronic device may further send a first request to the cloud server, where the first request is used to indicate the cloud server to determine care prompt information based on the ovarian health evaluation result, and send the care prompt information to the third electronic device.

Optionally, step S517: The cloud server determines the care prompt information based on the ovarian health evaluation result.

In some embodiments, after receiving the ovarian health evaluation result, the cloud server may determine the care prompt information based on the ovarian health evaluation result.

In some embodiments, after receiving the ovarian health evaluation result, the cloud server may determine the care prompt information based on the ovarian health evaluation result.

Optionally, step S518: The cloud server sends the care prompt information to the third electronic device based on the first relationship.

Optionally, step S519: The third electronic device receives the care prompt information, and the third electronic device displays a care prompt interface, where the care prompt interface includes the care prompt information.

Specifically, the first electronic device may further collaborate with a family device (namely, the third electronic device) to perform relative or friend care. For example, the first electronic device pushes, to a relative or friend, information indicating that physical discomfort, an emotional fluctuation, and the like may occur on the user, so that the relative or friend makes preparations in advance, provides more care, and avoids an emotional conflict, to reduce user discomfort. The family care prompt interface may be the user interface 40 mentioned above. For detailed descriptions of the user interface 40, refer to the foregoing descriptions of FIG. 5. Details are not described herein again. The family care prompt interface may be a fifth interface.

In some embodiments, the first electronic device may send the ovarian health evaluation result to a target electronic device in the N second electronic devices, where the target electronic device may be a smartphone, the target electronic device may obtain an application interface of a third-party application of the target electronic device, and may send the ovarian health evaluation result to the third-party application. In another embodiment, the first electronic device may also obtain an application interface of a third-party application of the first electronic device, and may send the ovarian health evaluation result to the third-party application. Further, the first electronic device may collaborate with the third-party application of the first electronic device and/or the third-party application of the second electronic device, and the third-party application may push a related product based on the ovarian health evaluation result. For example, TikTok pushes a short video of easy reservation, and Taobao pushes a product that can be used to relieve a related symptom.

In some embodiments, the first electronic device may send the ovarian health evaluation result to a target application. The target application may be a system application and/or a third-party application, and the target application presents the ovarian health evaluation result and/or the related detection data to a doctor with user's consent, to reduce consultation complexity of the user, and improve diagnosis accuracy and efficiency of the doctor, so as to implement an intelligent consultation service and improve user experience.

In some embodiments, the first electronic device may push content related to an assisted reproduction technology based on the ovarian health evaluation result, for example, provide assisted reproduction-related knowledge for a user who has a fertility requirement.

In some embodiments, the first electronic device may perform a part or all of step S508 to step S510 through the data obtaining module in FIG. 1c.

In some embodiments, the first electronic device may perform a part or all of step S511 through the ovarian function evaluation module in FIG. 1c.

In some embodiments, the first electronic device may determine the target data based on the user vital sign data through the signal processing and feature extraction module in FIG. 1c.

In conclusion, in this embodiment of this application, the first electronic device may receive the first operation (for example, a touch operation) performed by the user on the detection start control (namely, the first control) on the detection start interface (namely, the first interface), and obtain the first data. The first data includes at least the heart rate variability data of the user within the preset time period. The user vital sign data may include the first data. The user vital sign data may be used as a basis for evaluating ovarian health of the user, and the ovarian health evaluation result may be determined based on the user vital sign data. In this application, the ovarian health of the user can be evaluated based on the heart rate variability data of the user, and the ovarian health of the user does not need to be evaluated through endocrine detection, so that a portable and low-cost ovarian health evaluation solution is provided. Further, after the preset time period, a detection result interface may be displayed on the first electronic device, and display content of the detection result interface includes the ovarian health evaluation result. The ovarian health evaluation result of the user is displayed on the first electronic device, so that the abnormal ovarian function of the user can be detected in a timely manner. If the symptom of abnormal ovarian aging can be detected in a timely manner, a related symptom caused by ovarian aging can be improved.

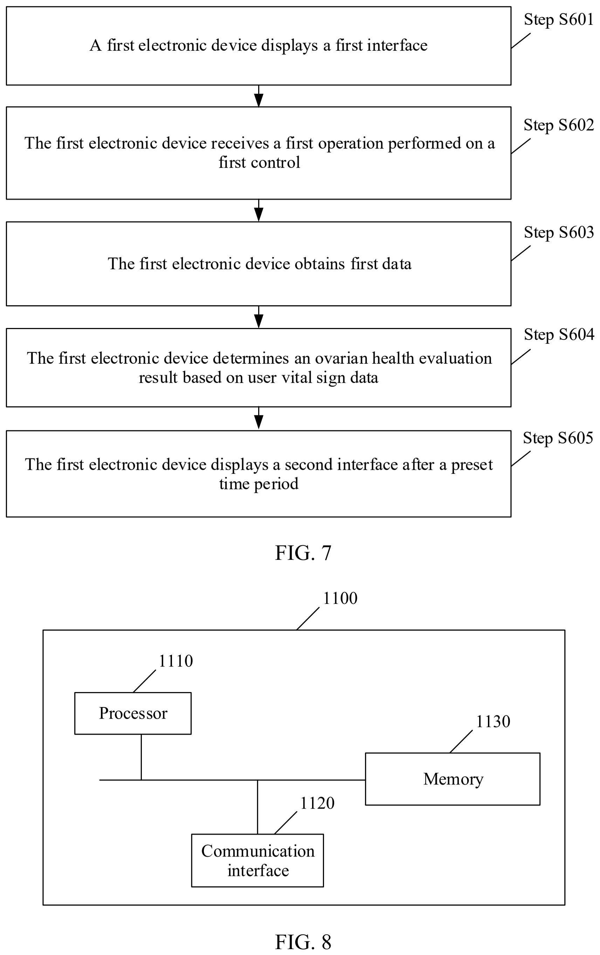

FIG. 7 is a schematic flowchart of another ovarian health evaluation method according to an embodiment of this application. The ovarian health evaluation method is applied to a first electronic device. Details are described below.

Step S601: The first electronic device displays a first interface.

Specifically, the first interface includes a first control. For specific implementation of step S601, refer to the detailed descriptions of step S507 in FIG. 6A to FIG. 6C. Details are not described herein again.

Step S602: The first electronic device receives a first operation performed on the first control.

Specifically, the first operation may be a touch operation, a sliding operation, a mid-air operation, or the like.

Step S603: The first electronic device obtains first data.

Specifically, the first data includes heart rate variability data of a user within a preset time period. For specific implementation of step S603, refer to the detailed descriptions of step S508 in FIG. 6A to FIG. 6C. Details are not described herein again.

Step S604: The first electronic device determines an ovarian health evaluation result based on user vital sign data.

Specifically, the user vital sign data includes first data, and the ovarian health evaluation result indicates an ovarian health status of the user within the preset time period. For specific implementation of step S604, refer to the detailed descriptions of step S510 and step S511 in FIG. 6A to FIG. 6C. Details are not described herein again.

Step S605: The first electronic device displays a second interface after the preset time period.

Specifically, display content of the second interface includes the ovarian health evaluation result. For specific implementation of step S605, refer to the detailed descriptions of step S512 in FIG. 6A to FIG. 6C. Details are not described herein again.

In conclusion, in this embodiment of this application, the first electronic device may receive the first operation (for example, a touch operation) performed by the user on a detection start control (namely, the first control) of a detection start interface (namely, the first interface), and obtain first user vital sign data. The first user vital sign data includes at least the heart rate variability data of the user within the preset time period. The user vital sign data may include the first user vital sign data. The user vital sign data may be used as a basis for evaluating ovarian health of the user, and the ovarian health evaluation result may be determined based on the user vital sign data. In this application, the ovarian health of the user can be evaluated based on the heart rate variability data of the user, and the ovarian health of the user does not need to be evaluated through endocrine detection, so that a portable and low-cost ovarian health evaluation solution is provided. Further, after the preset time period, a detection result interface may be displayed on the first electronic device, and display content of the detection result interface includes the ovarian health evaluation result. The ovarian health evaluation result of the user is displayed on the first electronic device, so that an abnormal ovarian function of the user can be detected in a timely manner. If a symptom of abnormal ovarian aging can be detected in a timely manner, a related symptom caused by ovarian aging can be improved, and female health can be improved.

This application provides a computer storage medium, where the computer storage medium stores a computer program, and when the computer program is executed by a processor, any one of the foregoing ovarian health evaluation methods is implemented.

FIG. 8 is a diagram of an electronic device according to an embodiment of this application. An embodiment of this application provides an electronic device 1100. The electronic device 1100 includes a processor 1110. The processor 1110 is configured to support the electronic device 1100 in implementing a corresponding function in any one of the foregoing ovarian health evaluation methods. The electronic device 1100 may further include a memory 1130. The memory 1130 is configured to: couple to the processor 1110, and store program instructions and data that are necessary for the electronic device 1100. The electronic device 1100 may further include a communication interface 1120, configured to perform communication between the electronic device 1100 and another device or a communication network.

This application provides a chip system. The chip system includes a processor, configured to support the electronic device in implementing the foregoing functions, for example, determine or process information related to the foregoing ovarian health evaluation method. In a possible design, the chip system further includes a memory. The memory is configured to store program instructions and data that are necessary for the electronic device. The chip system may include a chip, or may include a chip and another discrete component.

This application provides a computer program. The computer program includes instructions, and when the computer program is executed by a computer, the computer is enabled to perform the foregoing ovarian health evaluation method.

In the foregoing embodiments, the descriptions of each embodiment have respective focuses. For a part that is not described in detail in an embodiment, refer to related descriptions in other embodiments.

It should be noted that, for brief description, the foregoing method embodiments are represented as a series of actions. However, a person skilled in the art should appreciate that this application is not limited to the described order of the actions. Because according to this application, some steps may be performed in other orders or simultaneously. It should be further appreciated by a person skilled in the art that embodiments described in this specification all belong to example embodiments, and the involved actions and modules are not necessarily required by this application.

In the several embodiments provided in this application, it should be understood that the disclosed apparatus may be implemented in other manners. For example, the described apparatus embodiment is merely an example. For example, division into the units is merely logical function division and may be other division in actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electrical or other forms.

The units described as separate components may or may not be physically separate, and components displayed as units may or may not be physical units, that is, may be located at one position, or may be distributed on a plurality of network units. A part or all of the units may be selected as required to achieve the objectives of the solutions of embodiments.

In addition, function units in embodiments of this application may be integrated into one processing unit, each of the units may exist independently physically, or two or more units may be integrated into one unit. The integrated unit may be implemented in a form of hardware, or may be implemented in a form of software functional unit.

When the integrated unit is implemented in the form of software function unit and sold or used as an independent product, the integrated unit may be stored in a computer-readable storage medium. Based on such an understanding, the technical solutions of this application essentially, or the part contributing to the conventional technology, or all or some of the technical solutions may be implemented in a form of software product. The computer software product is stored in a storage medium and includes several instructions for instructing a computer device (which may be a personal computer, a server, a network device, or the like, and may be specifically a processor in a computer device) to perform all or some of the steps of the methods described in embodiments of this application. The foregoing storage medium includes any medium that can store program code, such as a USB flash drive, a removable hard disk, a magnetic disk, an optical disc, a read-only memory (Read-Only Memory, ROM for short), or a random access memory (Random Access Memory, RAM for short).

In conclusion, the foregoing embodiments are merely intended for describing the technical solutions of this application, but not for limiting this application. Although this application is described in detail with reference to the foregoing embodiments, a person of ordinary skill in the art should understand that modifications may still be made to the technical solutions described in the foregoing embodiments or equivalent replacements may still be made to some technical features thereof, without departing from the spirit and scope of the technical solutions of embodiments of this application.

**Claims**

1. An ovarian health evaluation method, applied to a first electronic device, wherein the method comprises:

   displaying a first interface, wherein the first interface comprises a first control;
   receiving a first operation performed on the first control;
   obtaining first data, wherein the first data comprises heart rate variability data of a user within a preset time period;
   determining an ovarian health evaluation result based on user vital sign data, wherein the user vital sign data comprises the first data, and the ovarian health evaluation result indicates an ovarian health status of the user within the preset time period; and
   displaying a second interface after the preset time period, wherein the second interface comprises the ovarian health evaluation result.

2. The method according to claim 1, wherein the first electronic device is connected to N second electronic devices, and N is an integer greater than 0; and after receiving the first operation performed on the first control, the method further comprises:
   receiving second data sent by M second electronic devices in the N second electronic devices, wherein M is an integer greater than 0 and less than or equal to N, and the user vital sign data further comprises the second data.

3. The method according to claim 2, wherein the second data comprises one or more of the following: blood pressure data of the user within the preset time period, body fat data of the user within the preset time period, emotion data of the user within the preset time period, and skin temperature data of the user within the preset time period.

4. The method according to any one of claims 1 to 3, wherein the method further comprises:

   displaying a third interface, wherein the third interface comprises a user questionnaire survey question; and
   receiving an input operation of the user, and determining questionnaire survey data, wherein the user vital sign data further comprises the questionnaire survey data.

5. The method according to any one of claims 1 to 4, wherein determining the ovarian health evaluation result based on the user vital sign data comprises:

   obtaining user information, wherein the user information comprises one or more of medication, drinking, a disease history, and a sleep environment temperature of the user; and
   determining the ovarian health evaluation result based on the user information and the user vital sign data.

6. The method according to any one of claims 1 to 5, wherein the first data further comprises one or more of the following: heart rate data of the user within the preset time period, respiratory rate data of the user within the preset time period, and the skin temperature data of the user within the preset time period.

7. The method according to claim 6, wherein the method further comprises:

   obtaining one or more of the heart rate data, the respiratory rate data, and the skin temperature data;
   displaying a fourth interface when one or more of the heart rate data, the respiratory rate data, and the skin temperature data meet a first condition, wherein the fourth interface comprises a third control and a fourth control, the third control is used by the user to confirm that a hot flash currently occurs, and the fourth control is used by the user to confirm that no hot flash currently occurs; and
   receiving an operation performed by the user on the third control or the fourth control.

8. The method according to claim 7, wherein the first condition is that a data change amplitude of one or more of the heart rate data, the respiratory rate data, and the skin temperature data in a first time interval within the preset time period is greater than or equal to a first preset threshold.

9. The method according to any one of claims 1 to 8, wherein determining the ovarian health evaluation result based on the user vital sign data comprises:

determining target data based on the user vital sign data; and

determining the ovarian health evaluation result based on the target data and an ovarian function evaluation model.

10. The method according to claim 9, wherein determining the target data based on the user vital sign data comprises:

determining symptom information of the user based on the user vital sign data, wherein the symptom information comprises a hot flash frequency and an autonomic nerve function level of the user within the preset time period; and determining the target data based on the symptom information.

11. The method according to claim 9 or 10, wherein the method further comprises:

obtaining user health check data, wherein the user health check data comprises a user anti-mullerian hormone test result and/or a user follicle-stimulating hormone test result; and adjusting a parameter in the ovarian function evaluation model based on the user health check data.

12. The method according to any one of claims 1 to 11, wherein the first data further comprises a sleep parameter of the user within the preset time period.

13. The method according to claim 12, wherein the N second electronic devices comprise a smart speaker, and the method further comprises:

determining, based on the sleep parameter within the preset time period, whether sleep quality of the user is less than a second preset threshold; and if the sleep quality is less than the second preset threshold, sending first information to the smart speaker, wherein the first information indicates the smart speaker to play a target audio.

14. The method according to any one of claims 2 to 13, wherein the N second electronic devices comprise a smart air conditioner, and the method further comprises:
predicting, within the preset time period, a target moment at which the user has a hot flash, and sending second information to the smart air conditioner before the target moment, wherein the second information indicates the smart air conditioner to adjust to a target temperature.

15. The method according to any one of claims 1 to 14, wherein the method further comprises:
uploading the ovarian health evaluation result to a cloud server, wherein the cloud server is configured to: determine care prompt information based on the ovarian health evaluation result, and send the care prompt information to a third electronic device, wherein the third electronic device and the first electronic device are bound as a first relationship; and the third electronic device is configured to display a fifth interface, wherein display content of the fifth interface comprises the care prompt information.

16. An electronic device, comprising a memory and one or more processors, wherein the memory is coupled to the one or more processors, the memory is configured to store computer program code, the computer program code comprises computer instructions, and the one or more processors invoke the computer instructions, to enable the electronic device to perform the method according to any one of claims 1 to 15.

17. A chip system, wherein the chip system comprises at least one processor, a memory, and an interface circuit, the memory, the interface circuit, and the at least one processor are interconnected through a line, the at least one memory stores instructions, and when the instructions are executed by the processor, the method according to any one of claims 1 to 15 is implemented.

18. A computer-readable storage medium, comprising instructions, wherein when the instructions are run on an electronic device, the electronic device is enabled to perform the method according to any one of claims 1 to 15.

Electronic device 100

FIG. 1a

| Application layer | **Huawei health** | Book | Music | Messages | | |
|---|---|---|---|---|---|---|
| | Gallery | Phone | Navigation | WLAN | Video | ... |

| Application framework layer | Window manager | Content provider | Phone manager | Resource manager |
|---|---|---|---|---|
| | Notification manager | View system | Event manager | ... |

| System library | Surface manager | Media library | **Ovarian health evaluation module** | Android runtime |
|---|---|---|---|---|
| | 2D graphics engine | 3D engine module | | ... |

| Kernel layer | Display driver | Camera driver | |
|---|---|---|---|
| | Audio driver | Sensor driver | ... |

FIG. 1b

Data obtaining module

↓

Signal processing and feature extraction module

↓

Ovarian function evaluation module

FIG. 1c

FIG. 2

First electronic device

20

201

FIG. 3a

First electronic device

Ovarian health evaluation

21

Detection start

211

FIG. 3b

First electronic device

22

Questionnaire survey

Your recent emotional
fluctuation frequency:

None

Occasional
(≤X times per day)

Often
(>X times per day)

FIG. 3c(a)

First electronic device

22

Questionnaire survey

Your recent bone
joint pain frequency:

None

Occasional
($\leq$y times per day)

Often
($>$y times per day)

FIG. 3c(b)

First electronic device

22

Questionnaire survey

Is your recent menstrual
period regular:

Yes

No

FIG. 3c(c)

First electronic
device

23

Ovarian health
evaluation result

The risk of the ovarian
function decline is low for
you. Please keep it

(a)

First electronic
device

23

Ovarian health
evaluation result

The risk of the ovarian
function decline is high for
you. Tap to view related
improvement suggestions

View

231

(b)

FIG. 3d

First electronic device

Knowledge push

Appropriate XX exercise, XX ingredients, and XX rest mode can improve your ovarian health

24

(a)

First electronic device

Smart home collaboration

The sleep-aiding audio is recommended. Play it for you?

Yes   No

24

(b)

First electronic device

Intelligent consultation

Your ovarian evaluation result information may be helpful for consultation. Do you want to share it with your doctor?

Yes   No

24

(c)

FIG. 3e

First electronic device

25 — It is detected that your body temperature and heart rate fluctuate significantly in the past XX minutes. Do you have a hot flash?

Yes

No

FIG. 3f

First electronic device

26 — Evaluating...

FIG. 3g

First electronic device

27

Hot flash frequency:
10 times/day

Quantity
of hot
flashes

20
15
15
10
10
10
5
5
5

First
day

Second
day

Third
day

Fourth
day

(a)

First electronic device

27

Sleep status indicator:
Excellent

Sleep
score

90
91
81
78
100
80
60
40

First
day

Second
day

Third
day

Fourth
day

(b)

FIG. 3h

EP 4 730 357 A1

FIG. 3i

FIG. 4a

5G|||| 5G|||| 📶      🔋 8:00

← **Huawei health**

User interface 31

Steps

Heart rate

3011 — Ovarian health evaluation

Exercise duration

3012 — Medication setting

3013 — Health check report input

FIG. 4b

User interface 32

Ovarian health evaluation

Ovarian health evaluation result: high risk

Push

**Diet collocation**

FIG. 4c

5G ▮▮▮▮ 5G ▮▮▮▮ 📶　　　　　🔋 8:00

My home >

330

User interface 33

8 devices

| | |
|---|---|
| **Television in the living room** Online (living room) | **Television in the master bedroom** Offline (master bedroom) |
| **Air conditioner in the living room** Online (living room) | **Speaker in the living room** Online (living room) |
| **Speaker in the master bedroom** Online (master bedroom) | **Blood pressure monitor** Online  High pressure: 120 Low pressure: 70 |
| **Body fat scale** Online | **Huawei router** Online (living room) |

Home　　Store　　Intelligence　　Me

FIG. 4d

5G .ıll 5G .ıll 🛜                    ▬ 8:00

← **Ovarian health evaluation**

User interface 32

Ovarian health risk

   No data available

Compared with a peer group

Ovarian health
  degree

| Young<br>Low-risk area | Old<br>Low-risk area |
|---|---|
| | Average line |
| Young<br>High-risk area | Old<br>High-risk area |

                40 years old       Age

FIG. 4e(a)

**Ovarian health evaluation**

User interface 32

Ovarian health risk

Low risk
▼

Result interpretation
It is found that the risk of premature ovarian failure is low and recommended to avoid sitting for a long time, avoid eating more stimulating food, and keep a good mentality

Compared with a peer group
Ovarian health degree

Young
Low-risk area
Your location

Old
Low-risk area

Average line

Young
High-risk area

Old
High-risk area

40 years old
Age

8:00

FIG. 4e(b)

Third electronic device

40

Relative and friend care

Your family XX may have a large
emotional fluctuation recently.
Please pay appropriate attention

FIG. 5

| First electronic device | N second electronic devices | Third electronic device | Cloud server |
|---|---|---|---|

Optionally, step S501: The first electronic device establishes a connection to the N second electronic devices

Optionally, step S502: The first electronic device sends a binding request to the cloud server

Optionally, step S503: The cloud server sends binding confirmation information to the third electronic device

Optionally, step S506: The first electronic device displays an application interface, where the application interface includes an ovarian health application control

Optionally, step S504: The third electronic device sends confirmation binding information to the cloud server

Step S507: The first electronic device receives an operation performed by a user on the ovarian health application control on the application interface, and displays a detection start interface of an ovarian health evaluation application, where the detection start interface includes a detection start control

Optionally, step S505: The cloud server sets the first electronic device and the third electronic device to a first relationship

Optionally, step S508: The first electronic device receives a first operation performed by the user on the detection start control on the detection start interface, and obtains first data

TO
FIG. 6B

TO
FIG. 6B

TO
FIG. 6B

TO
FIG. 6B

FIG. 6A

CONT. FROM FIG. 6A        CONT. FROM FIG. 6A        CONT. FROM FIG. 6A        CONT. FROM FIG. 6A

Optionally, step S509: The first electronic device obtains second data from M second electronic devices in the N second electronic devices

Step S510: The first electronic device determines user vital sign data based on the first data

Step S511: The first electronic device determines an ovarian health evaluation result based on the user vital sign data

Step S512: After a preset time period, the first electronic device receives the operation performed by the user on the ovarian health application control on the application interface, and displays a detection result interface, where display content of the detection result interface includes the ovarian health evaluation result

Optionally, step S513: The first electronic device displays a knowledge push interface, where display content of the knowledge push interface includes knowledge push content determined based on the ovarian health evaluation result

Optionally, step S514: When the first electronic device predicts that the user has a hot flash or detects that sleep quality of the user is poor, the first electronic device sends corresponding information to a part or all of the N second electronic devices, where the information indicates the part or all of the second electronic devices to adjust a space environment in which the user is located

TO FIG. 6C        TO FIG. 6C        TO FIG. 6C        TO FIG. 6C

FIG. 6B

CONT.
FROM
FIG. 6B

CONT.
FROM
FIG. 6B

CONT.
FROM
FIG. 6B

CONT.
FROM
FIG. 6B

Optionally, step S515: The part or all of the N second electronic devices receive a request for adjusting the user space environment, and adjust the user space environment

Optionally, step S516: The first electronic device uploads the ovarian health evaluation result to the cloud server

Optionally, step S517: The cloud server determines care prompt information based on the ovarian health evaluation result

Optionally, step S518: The cloud server sends the care prompt information to the third electronic device based on the first relationship

Optionally, step S519: The third electronic device receives the care prompt information, and the third electronic device displays a care prompt interface, where the care prompt interface includes the care prompt information

FIG. 6C

A first electronic device displays a first interface — Step S601

The first electronic device receives a first operation performed on a first control — Step S602

The first electronic device obtains first data — Step S603

The first electronic device determines an ovarian health evaluation result based on user vital sign data — Step S604

The first electronic device displays a second interface after a preset time period — Step S605

FIG. 7

1100

1110

Processor

1130

Memory

1120

Communication interface

FIG. 8

## INTERNATIONAL SEARCH REPORT

| International application No. |
| --- |
| **PCT/CN2024/144183** |

| A. | CLASSIFICATION OF SUBJECT MATTER |
| --- | --- |

G16H50/30(2018.01)i; A61B5/0205(2006.01)i

According to International Patent Classification (IPC) or to both national classification and IPC

| B. | FIELDS SEARCHED |
| --- | --- |

Minimum documentation searched (classification system followed by classification symbols)

IPC: G16H, A61B

Documentation searched other than minimum documentation to the extent that such documents are included in the fields searched

Electronic data base consulted during the international search (name of data base and, where practicable, search terms used)

CNTXT, ENTXT, ENTXTC, VEN: 心率, 变异, 卵巢, 黄体, 更年期, 健康, 评估, 血压, 体脂, 情绪, 体温, 显示, 界面, 穿戴, 手环, 潮热, 空调, 自主神经, ovary, HRV, blood pressure, fat, temperature, display, flush

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | CN 116195982 A (HUAWEI TECHNOLOGIES CO., LTD.) 02 June 2023 (2023-06-02) claims 1, 5-6 and 11-13, and description, paragraphs 42, 45 and 79 | 1-18 |
| Y | CN 116726470 A (HONOR DEVICE CO., LTD.) 12 September 2023 (2023-09-12) claim 1 | 1-18 |
| Y | CN 110236489 A (HUAWEI TECHNOLOGIES CO., LTD.) 17 September 2019 (2019-09-17) claims 1-18 | 1-18 |
| Y | CN 115530768 A (HUAWEI TECHNOLOGIES CO., LTD.) 30 December 2022 (2022-12-30) claims 1-19 | 1-18 |
| Y | CN 109308938 A (SHANGHAI IBABY YIJIA NETWORK TECHNOLOGY CO., LTD.) 05 February 2019 (2019-02-05) claim 1 | 3 |
| Y | WO 2022056306 A1 (WEBSTER, Wade W.) 17 March 2022 (2022-03-17) claims 1 and 4-5 | 6-8 |

| ✓ Further documents are listed in the continuation of Box C. | ✓ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **27 March 2025** | **02 April 2025** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2024/144183** |

### C.    DOCUMENTS CONSIDERED TO BE RELEVANT

| Category* | Citation of document, with indication, where appropriate, of the relevant passages | Relevant to claim No. |
|---|---|---|
| Y | WO 2022212741 A1 (OURA HEALTH OY) 06 October 2022 (2022-10-06)<br>description, paragraph 23 | 6-8 |
| Y | JP 2000140117 A (SHISEIDO CO., LTD.) 23 May 2000 (2000-05-23)<br>description, paragraph 32 | 12-13 |
| Y | CN 116322898 A (AMIRA HEALTH, INC.) 23 June 2023 (2023-06-23)<br>description, paragraph 98 | 14 |
| A | CN 113130077 A (WANG SHIXUAN) 16 July 2021 (2021-07-16)<br>description, paragraph 60 | 1-18 |
| A | CN 114300088 A (SECOND AFFILIATED HOSPITAL OF SOOCHOW UNIVERSITY) 08<br>April 2022 (2022-04-08)<br>description, paragraphs 2-20 | 1-18 |
| A | CN 116705327 A (KANGJIAN INFORMATION TECHNOLOGY (SHENZHEN) CO.,<br>LTD.) 05 September 2023 (2023-09-05)<br>description, paragraphs 78-81 | 1-18 |
| A | JP 2015019753 A (UNIVERSITY OF OCCUPATIONAL AND ENVIRONMENTAL<br>HEALTH, JAPAN) 02 February 2015 (2015-02-02)<br>description, paragraphs 1-3 | 1-18 |
| A | US 2020410891 A1 (COGNIFISENSE, INC.) 31 December 2020 (2020-12-31)<br>description, paragraph 21 | 1-18 |

Form PCT/ISA/210 (second sheet) (July 2022)

## INTERNATIONAL SEARCH REPORT
### Information on patent family members

International application No.

**PCT/CN2024/144183**

| Patent document cited in search report | | | Publication date (day/month/year) | Patent family member(s) | | | Publication date (day/month/year) |
|---|---|---|---|---|---|---|---|
| CN | 116195982 | A | 02 June 2023 | None | | | |
| CN | 116726470 | A | 12 September 2023 | None | | | |
| CN | 110236489 | A | 17 September 2019 | None | | | |
| CN | 115530768 | A | 30 December 2022 | None | | | |
| CN | 109308938 | A | 05 February 2019 | None | | | |
| WO | 2022056306 | A1 | 17 March 2022 | None | | | |
| WO | 2022212741 | A1 | 06 October 2022 | CA | 3215586 | A1 | 06 October 2022 |
| | | | | JP | 2024514495 | A | 02 April 2024 |
| | | | | AU | 2022249145 | A1 | 12 October 2023 |
| JP | 2000140117 | A | 23 May 2000 | None | | | |
| CN | 116322898 | A | 23 June 2023 | WO | 2022026858 | A1 | 03 February 2022 |
| | | | | US | 2023226308 | A1 | 20 July 2023 |
| | | | | EP | 4178654 | A1 | 17 May 2023 |
| | | | | EP | 4178654 | A4 | 07 August 2024 |
| CN | 113130077 | A | 16 July 2021 | None | | | |
| CN | 114300088 | A | 08 April 2022 | None | | | |
| CN | 116705327 | A | 05 September 2023 | None | | | |
| JP | 2015019753 | A | 02 February 2015 | JP | 6281892 | B2 | 21 February 2018 |
| US | 2020410891 | A1 | 31 December 2020 | WO | 2019183129 | A1 | 26 September 2019 |
| | | | | EP | 3782160 | A1 | 24 February 2021 |
| | | | | EP | 3782160 | A4 | 15 December 2021 |

Form PCT/ISA/210 (patent family annex) (July 2022)

**EP 4 730 357 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202410015268

**53**